# EUROPEAN PATENT SPECIFICATION

(11) **EP 2 430 057 B1**
(45) Date of publication and mention of the grant of the patent: **09.10.2013**
(21) Application number: 10720665.8
(22) Date of filing: 14.05.2010
(51) Int. Cl.: C08F 220/56, C08F 220/26, C09D 133/26, C09D 143/04, C12N 1/02, C12N 11/08, C12N 5/00

(54) **TEMPERATURE-RESPONSIVE CO-POLYMERS AND USES THEREOF**
TEMPERATUREMPFINDLICHE CO-POLYMERE UND DEREN VERWENDUNGEN
COPOLYMÈRES SENSIBLES À LA TEMPÉRATURE ET UTILISATIONS DE CEUX-CI

(30) Priority: 15.05.2009 GB 0908372
(43) Date of publication of application: 21.03.2012
(73) Proprietor: The University Of Manchester, Manchester M13 9PL (GB)
(72) Inventor: LU, Jian, Manchester M13 9PL (GB); YANG, Lei, Manchester M13 9PL (GB)
(74) Representative: Harrison Goddard Foote LLP
(86) International application number: PCT/GB2010/050789
(87) International publication number: WO 2010/131048

(56) References cited:
- EP-A1- 1 873 205
- WO-A1-2005/007717
- WO-A1-2008/004988
- WO-A2-2004/011669

## Description

This invention relates to temperature-responsive co-polymers, polymeric films formed from these co-polymers and particular applications of these polymeric films, particularly in the cell culture field. More specifically, the present invention relates to the use of these polymeric films in the cell culture field to provide a means of controlling cellular adhesion to, and detachment from, the surface of a cell culture support substrate.

### BACKGROUND

The ability to grow eukaryotic and prokaryotic cells in culture *in vitro* is utilised in a wide range of commercial and/or research applications.

Certain cell types can be grown in suspension cultures, where the cells are effectively dispersed or "floating" within a suitable growth medium, whereas other cell types, including many mammalian cells, will only grow and proliferate in a culture medium once they have adhered to the surface of suitable support substrate (such as, for example, one of the internal surfaces of a cell culture flask or plate).

In order to maintain the long term viability of cells grown in culture and avoid the senescence associated with a high cell density within a culture medium, it is necessary to passage (or "split") the cells on a regular basis. This process involves harvesting a small number of cells from the mature culture medium and seeding them into a fresh culture medium in which they can continue to grow and remain viable. It is also necessary to be able to harvest the cells from a culture medium so that they can be used for a particular application. In the case of suspension cultures, the cells can be harvested by simply removing some or all of the mature suspension culture medium. However, in the case of cells which grow on the surface of a support substrate, such as an internal surface of a cell culture plate or flask, the cells will be adhered to the support surface and will need to be dislodged, either by mechanical agitation (e.g. scraping or vigorous and repeated flushing of the support surface with fluid) and/or by using enzymes (e.g. trypsin) to digest components of the extracellular matrix which bind the cells to the support surface. One problem associated with these enzymatic and/or mechanical techniques is that they not only invasively destroy cell-to-cell junction proteins leading to cell release, but also irreversibly damage cell membrane-associated proteins resulting in the loss of some cell function (Yamato et al., J. Biomed. Mater. Res., 1999, 44: 44-52; Shimizu et al., Biomaterials, 2003, 24: 2309-2316; Canavan et al., J Biomed Mater Res, 2005, 75A: 1-13).

Accordingly, there is a need for alternative and improved approaches to dislodge cells from the surface of a support substrate.

One alternative approach involves the use of a temperature-responsive polymer, such as poly(N-isopropylacrylamide) (PNIPAAm) for cell harvesting (Yamada et al., Makromol. Chem. Rapid Commun., 1990, 11: 571-576). PNIPAAm and its derivatives exhibit a lower critical solution temperature (LCST) in aqueous solution (Kwon et al., Biomaterials, 2003, 24:1223-1232). Across the LCST, the polymer shows remarkable changes in hydration in response to small variations in temperature. The PNIPAAm chains become hydrated to form an expanded structure below LCST, while above the LCST, the polymer chains are dehydrated and become structurally condensed. This temperature-responsive behaviour is reversible and is associated with the formation and deformation of hydrogen bonds between water molecules and amide linkages. In addition to its use in the cell culture field, temperature-responsive PNIPAAm has been studied for a number of different applications, including drug delivery and the immobilisation and separation of proteins.

In cell culture, a thin PNIPAAm film is usually grafted or coated onto culture plates or commercial membranes. Cells are mostly cultured at around 37°C *in vitro.* At this temperature, the polymer film is dehydrated and its surface is primarily hydrophobic and well suited for cell attachment and growth. Upon reaching confluence, the temperature is reduced to below 30°C and the thin film becomes hydrated. Upon hydration, the film volume expands and its surface becomes hydrophilic. The combination of the expansion of the polymer film and its hydrophilic nature force cells to detach from the substrate. The use of a PNIPAAm temperature-responsive polymer to remove adhered cells possesses a number of advantages relative to the most commonly used enzymatic digestion or mechanical dissociation techniques. In particular, as the cells grown on PNIPAAm coated substrates are recovered by just a drop in temperature the cell membranes of the cells remain intact. Consequently, the cells retain better growth activities and biological function when compared to those harvested using enzymes or cell scrapers (Canavan et al., J Biomed Mater Res, 2005, 75A: 1-13).

However, despite the benefits offered by the temperature-responsive PNIPAAm for cell culture, its use commercially has remained limited. This is primarily because most PNIPAAm films that have been fixed onto culture plates or membranes are generated through elaborate and expensive treatment procedures such as electron beam irradiation (Yamada et al., Makromol.Chem.Rapid Commun, 1990, 11: 571-576; Kwon et al., Biomaterials, 2003, 24:1223-1232; Yamato et al., J Biomed Mater Res, 1999, 44: 44-52; Shimizu et al., Biomaterials, 2003, 24: 2309-2316), plasma treatment (Akiyama et al, Langmuir, 2004, 20: 5506-5511; Schmaljohann et al., Biomacromolecules, 2003, 4: 1733-1739; Nitschke et al., Express Polymer Letters, 2007, I: 660-666), UV irradiation (Chen et al., Materials Science and Engineering, 2005, 25: 472-478; Matsuda et al., Biomacromolecules 2007, 8: 2345-2349) and demanding chemistry approaches (Cho et al., Biomaterials, 2004, 25: 5743-5751; Ista et al., Langmuir, 2001, 17: 2552-2555). In each of these reactions, surfaces are activated and monomers are added into the solution. Polymerisation occurs and surface grafted polymer chains form from monomers grafted directly onto the substrate surface. The process of direct polymerisation of the temperature-responsive film onto substrate surface is laborious, time-consuming and expensive as it often involves expensive instrumentation and a laboratory environment. Accordingly, this has prevented the wide application of this technique.

Processes for the live sequential surface initiated atom transfer radical polymersization (SI-ATRP) to make a surface grafted block copolymer such as PNIPAAm-PS for cell attachment and detachment have also been described (Wu et al., Langmuir 2009, 25, 2900-2906 and Yu et al., Langmuir 2010, DOI: 10.1021/la904663m). However, the substrate silicon oxide surface needs to be grafted with an underlayer of 3-Aminopropyltriethoxysilane (APTES), followed by the chemical grafting of surface initiator such as bromoisobutyryl bromide (BIBB) or chloroacetyl chloride. As with other polymerisation approaches, this method also requires surface film formation *in situ* on the substrate surface. Furthermore, as many synthetic steps are involved, it is difficult to control surface film growth, causing uncertainty in predicting layer thickness and composition. Such processes will be difficult on a large scale.

The present invention seeks to address these drawbacks by providing a polymeric film coating for cell culture support substrates that is simple to make, inexpensive and reliable.

Accordingly, it is an object of the present invention to provide a simple, inexpensive and reliable means for providing a temperature-responsive polymeric film coating on the cell culture support substrate, which provides a surface upon which cells can grow and proliferate at the incubation temperature, but which hydrate and dislodge the cells when the temperature is reduced to below the incubation temperature.

### BRIEF SUMMARY OF THE DISCLOSURE

The present invention makes use of the temperature-responsive behavior of polymers comprising temperature-responsive monomeric units, such as N-isopropylacrylamide, to provide film coatings for cell culture support substrates. The polymeric film coating of the present invention provides a temperature dependent means of controlling cellular adhesion to, and detachment from, the surface of a cell culture support substrate. The novel temperature-responsive polymers of the present invention can be easily synthesised using well established polymerisation techniques. In addition, the polymers can be easily applied to, and immobilised on, the surface of a cell culture substrate to form the temperature dependent film coating.

The temperature responsive polymers of the present invention are co-polymers that comprise a mixture of temperature responsive monomers and additional hydrophobic and/or hydrophilic monomers. The co-polymers of the present invention also comprise a cross-linking means to enable the co-polymer to be covalently bound to the substrate surface to prevent detachment during use and to provide cross-links within the film to enhance its strength and integrity.

According to the present invention, there is provided a temperature-responsive co-polymer, a process, a substrate, a coating solution, a kit of parts, a method of forming a temperature-responsive film coating on a substrate surface, a method of dislodging cells, and a method of culturing cells as set forth in the appended claims. Optional features of the invention are evident from the dependent claims and the description that follows.

In a first aspect, the present invention provides a temperature-responsive co-polymer for forming a film coating on the surface of a cell culture substrate, the co-polymer comprising temperature-responsive hydrophilic monomeric units, non-temperature responsive hydrophobic and/or hydrophilic monomeric units and a cross-linking means to covalently bind the co-polymer to the substrate surface.

The properties of the co-polymer of the invention are selected so that it forms films which, at the incubation temperature, typically at or around 37°C for mammalian cells, are dehydrated and provide a hydrophobic surface on which the cells can adhere and grow and proliferate. However, upon cooling, the temperature responsive constituent of the co-polymer hydrates and swells. Without wishing to be bound by any particular theory, it is believed that the hydration and expansion of the polymeric film coating, together with its hydrophilic character when it is hydrated, causes the cells to be dislodged from the surface.

In a further aspect, the present invention provides a process for the preparation of a temperature-responsive co-polymer as defined herein, the process comprising the polymerisation of temperature-responsive monomeric units and one or more additional hydrophobic and/or hydrophilic monomeric units.

In a further aspect, the present invention provides a substrate having a surface upon which cells can grow and divide in culture, wherein said surface is coated with a polymeric film coating formed from a temperature responsive co-polymer as defined herein and said polymeric film coating is covalently bound to the substrate surface.

In a further aspect, the present invention provides a cell culture vessel comprising a substrate as defined herein.

In a further aspect, the present invention provides a coating solution for coating the surface of a substrate, the coating solution comprising a temperature-responsive co-polymer as defined herein dissolved in a suitable solvent.

In a further aspect, the present invention provides a kit of parts comprising a temperature responsive co-polymer as defined herein and a suitable solvent for dissolving the co-polymer to form a solution which can subsequently be applied to the substrate surface.

In a further aspect, the present invention provides a method of forming a temperature-responsive polymeric film coating on a substrate surface, said method comprising dissolving a temperature responsive co-polymer as defined herein in a suitable solvent to form a solution, applying the solution onto the surface of the substrate, removing the solvent and annealing the co-polymer to the substrate surface.

In a further aspect, the present invention provides a method of dislodging cells from a substrate surface which is coated with a temperature responsive co-polymer as defined herein, the method comprising cooling the substrate such that the temperature responsive co-polymer hydrates and thereby causes the cells present on the substrate surface to be dislodged.

In a further aspect, the present invention provides a method of culturing cells comprising seeding cells on a substrate as defined herein in the presence of a culture medium and maintaining the temperature at an incubation temperature to allow the cells to grow and divide and then reducing the temperature to harvest the cells.

### BRIEF DESCRIPTION OF THE DRAWINGS

Particular embodiments of the invention are further described hereinafter with reference to the accompanying drawings, in which:
Figure 1 is a diagram showing an exemplary apparatus for the formation of a PNIPAAm co-polymer according to the present invention;
Figure 2 is an illustration of the molecular structure of a PNIPAAm co-polymer of the present invention showing the possibilities for covalent bond formation (by methanol removal) when the co-polymer film is annealed (x: y: z = 20:1:1);
Figure 3 shows a FTIR spectrum for PNIPAAm co-polymer;
Figure 4 shows graphical representations of the hydrodynamic diameters of PNIPAAm co-polymers in UHQ (Elgastat ultrapure) water distribution by volume measured by DLS (dynamic light scattering) at different temperatures;
Figure 5 is graph showing the change in the hydrodynamic diameters of the PNIPAAm co-polymers in UHQ water measured by DLS at different temperatures;
Figure 6 is a graph showing the thickness changes of PNIPAAm co-polymer film (10mg/ml) while cooling down to 20°C and heating up to 37°C, repeatedly;
Figure 7 is a graph showing the thickness changes of PNIPAAm co-polymer film (5mg/ml) while cooling down to 20°C and heating up to 37°C repeatedly;
Figure 8 is a graph showing the water contact angles of dry and wet PNIPAAm co-polymer films and bare glass cover slips at 20°C and 37°C respectively;
Figure 9 shows the AFM topography (left) and phase (right) images of PNIPAAm co-polymer films with different concentrations - (A, B): 0.2mg/ml, 1µlm scan; (C, D): 1 mg/ml, 1 µm scan; (E, F): 5mg/ml, 2µm scan; (G, H): 10mg/ml, (10µm scan);
Figure 10 shows microscopic images of the adhesion at 37°C (left) and detachment at 20°C (right) of HeLa cells seeded on two different thickness films coated at 1 (A,B) and 5 (C,D) mg/ml co-polymer concentrations (scale bar left: 500 µm, right: 100µm);
Figure 11 shows microscopic images of the adhesion above LCST and detachment below LCST of HEK293 cells seeded on different thicknesses of PNIPAAm terpolymer films (scale bar: 500µm);
Figure 12 shows microscopic images of the adhesion above LCST and detachment below LCST of MSCs seeded on different thicknesses of PNIPAAm terpolymer films (scale bar: 100µm (left), 500µm (right));
Figure 13 shows microscopic images of the time-lapsed images of detachment of MSCs seeded below LCST on the optimal PNIPAAm co-polymer films (1mg/ml) (scale bar: 500µm);
Figure 14 shows microscopic images of the time-lapsed images of detachment of HEK293 cells seeded below LCST on the optimal PNIPAAm co-polymer films (2mg/ml)(scale bar: 500µm);
Figure 15 is a diagram illustrating the dislodgement of cells from a glass coverslip;
Figure 16 shows microscopic images of the growth (left) at 37°C and detachment (right) at 20°C of HeLa cells seeded on the optimal PNIPAAm co-polymer films (5mg/ml) in different cell culture cycles (scale bar: 100µm);
Figure 17 shows microscopic images of the growth (left) at 37°C and detachment (right) at 20°C of HEK293 cells seeded on the optimal PNIPAAm co-polymer films (2mg/ml) in different cell culture cycles (scale bar: 100µm);
Figure 18 shows microscopic images of the growth (left) at 37°C and detachment (right) at 20°C of BMMSCs seeded on the optimal PNIPAAm co-polymer films (1 mg/ml) in different cell culture cycles (scale bar: 100µm);
Figure 19 shows cell growth curves plotted as a function of increase in optical density (OD) measured from MTT assays (on the Y-axis) against time (X-axis) for human cardiac myocytes (HCM) grown on PNIPAAm coated coverslips against various control surfaces: plastic+film (●) denoting thermo-responsive film coated from 1 mg/ml copolymer solution, plastic (Δ) denoting TPCS surface, glass (□) and plastic+lysine (x) denoting bare TPCS surface adsorbed with a layer of polylysine. Each set of data was taken from the average of 3-4 duplicates;
Figure 20 shows cell growth curves plotted as a function of increase in optical density (OD) measured from MTT assays (on the Y-axis) against time for 3T3 fibroblast cells grown on PNIPAAm coated coverslips (1 mg/ml and 2 mg/ml) against various control surfaces: bare TPCS plastic (◆), glass (○), surfaces coated at 1 mg/ml (▲) and 2 mg/ml (■) and plastic+PL (●) denoting bare TPCS surface adsorbed with a layer of polylysine;
Figure 21 shows the monomeric components of the PNIPAAm/dodecylmethacrylate co-polymer with x:y:z:m = 16:1:1:2.

### DETAILED DESCRIPTION

The term "temperature-responsive" is used herein to refer to polymers that undergo a temperature dependent change in hydration. The temperature at which a substantial change in polymeric hydration occurs is known as the critical solution temperature (CST). The lower critical solution temperature (LCST) is the critical temperature below which the co-polymer becomes highly miscible with water and, in some cases, completely soluble. Accordingly, above the LCST the co-polymer is highly dehydrated and below the LCST the co-polymer is highly hydrated. Suitable polymers of the present invention have an LCST within the range of 10°C to 40°C. Especially suitable polymers possess a LCST within the range of 20°C to 35°C. The term "temperature-responsive" is also used herein to refer to monomers which, when polymerised, form temperature-responsive polymers that undergo a temperature dependent change in hydration as discussed above.

The term "alkyl", unless otherwise specified, is used herein to refer to linear or branched alkyl chains having 1 to 20 carbon atoms.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

### Temperature-responsive co-polymers

The present invention provides a temperature-responsive co-polymer for forming a film coating on the surface of a cell culture substrate, the co-polymer comprising temperature-responsive hydrophilic monomeric units, hydrophobic and/or hydrophilic monomeric units, and a cross-linking means to covalently bind the co-polymer to the substrate surface. The properties of the co-polymer of the invention are selected so that at the incubation temperature, typically at or around 37°C for mammalian cells (i.e. between 35°C and 39°C), the co-polymer is dehydrated and provides a hydrophobic surface upon which the cells can adhere to and grow and proliferate. However, upon cooling, the temperature responsive constituent of the co-polymer swells and hydrates and this presents a hydrophilic surface to the cells present on the surface. It is believed that the hydration and expansion of the polymeric film coating together with its hydrophilic character cause the cells to be dislodged from the surface.

Suitably, the co-polymers of the present invention are random co-polymers formed by simply mixing all of the monomeric components together in the desired proportions and carrying out the polymerisation.

The co-polymers of the invention may have any suitable polymeric backbone structure.

In particular embodiment of the invention, the polymeric backbone is formed by the polymerisation of substituted methacrylic, acrylic and/or bisacrylamide monomers.

In particular embodiment of the invention, the polymeric backbone is formed by the polymerisation of substituted methacrylic and/or acrylic monomers.

In an alternative embodiment, monomers such as methylenebisacrylamide, ethylenebisacrylamide, dioxyethylenebisacrylamide may be incorporated into the co-polymer to introduce amide bonds into the polymer backbone.

Suitably, the molecular weight of the temperature-responsive co-polymer is controlled. This enables the convenient removal of impurities following the polymerization reaction (e.g. unreacted monomers and initiator fragments) to provide a substantially pure co-polymer product, and also aids the film coating process, in particular the formation of uniform and reproducible co-polymer films. In an embodiment of the invention, the molecular weight of the co-polymer ranges from 10kD to 500kD. In a further embodiment of the invention, the molecular weight of the co-polymer ranges from 15kD to 300kD. In yet another embodiment, the molecular weight of the co-polymer ranges from 20kD to 100kD.

In a particular embodiment, the LCST of the co-polymer is between 25°C and 35°C. In a further embodiment, the LCST is between 28°C and 35°C. In yet another embodiment, the LCST is at or about 30°C. Such embodiments are particularly suitable for mammalian cell culture applications, where the cells are typically cultured at incubation temperatures of around 37°C.

Suitably, the temperature-responsive monomer is the main component of the polymer. Suitably, the molar fraction of the temperature-responsive monomer is greater than 0.3, and more typically it will be greater than 0.5. In an embodiment of the invention, the molar fraction of the temperature-responsive monomer is greater than 0.3 but less than 0.95. In a further embodiment, the molar fraction of the temperature-responsive monomer is greater than 0.5 but less than 0.95. In another embodiment, the molar fraction the temperature-responsive monomer is greater than 0.75 but less than 0.95.

Any suitable temperature-responsive monomers which, upon polymerisation, produce a co-polymer as defined herein may be used.

Particular examples of suitable temperature-responsive monomers include N-substituted alkyl acrylamides and N-substituted alkyl methacrylamides such as N-isopropylacrylamide and N-isopropylmethacrylamide, N,N-di-substituted alkyl acrylamide and N,N-di-substituted alkyl methacrylamide such as N,N-isopropylacrylamide and N,N-isopropylmethacrylamide and monomers containing ethylene oxide and propylene oxide units can also be used. Further examples of suitable temperature-responsive polymers are listed in WO2007/144356 (EP 1873205) at page 6, lines 9 to 18.

In an embodiment of the invention, the temperature responsive monomers are selected from N-alkyl substituted acrylamides and N-alkyl substituted methacrylamides such as N-isopropylacrylamide and N-isopropylmethacrylamide, N,N-di-alkyl substituted acrylamides and N,N-di-alkyl substituted methacrylamides such as N,N-isopropylacrylamide and N,N-isopropylmethacrylamide, or mixtures thereof. The alkyl groups may comprise 1 to 20 carbon atoms suitably 1 to 16 carbons atoms, and even more suitably 1 to 10 carbon atoms. The nature of the alkyl chain (i.e. its length and whether or not it is branched) and whether the acryamide or methacrlamide monomer is mono- or di- substituted will influence the nature of the polymeric film formed from the co-polymer and its LCST. A person skilled in the art will be able to select the appropriate number of carbon atoms for the alkyl group in order to modify the properties of the co-polymer film formed from the co-polymer.

In a further embodiment of the invention, the temperature-responsive monomer is selected from N-isopropylacrylamide and N-isopropylmethacrylamide, or a mixture thereof.

In a further embodiment of the invention, the temperature-responsive monomer is N-isopropylacrylamide.

Temperature sensitive hydrophobic monomers also include acrylamide and methacrylamide monomers substituted with one or more hydrophobic amino acids, such as alanine, valine and leucine.

The additional hydrophobic and/or hydrophilic monomeric components of the copolymer are necessary to adjust the hydrophilicity/hydrophobicity of the polymer, as well as the structure and integrity of the film coating ultimately formed by the polymer. This enables the surface properties and structure of the co-polymer film to be optimized for cell adhesion and detachment. These additional hydrophobic and/or hydrophilic monomeric components are not significantly temperature responsive below 40°C, and are therefore regarded as being "non-temperature responsive".

Any additional hydrophobic and/or hydrophilic monomers will typically be present in smaller amounts relative to the temperature responsive monomers. In a particular embodiment, the molar fraction of the additional hydrophobic and/or hydrophilic monomers is within the range 0.01 to 0.5. In a further embodiment of the invention, the molar fraction of the additional hydrophobic and/or hydrophilic monomers is within the range 0.01 to 0.2. In a further embodiment, the molar fraction of the additional hydrophobic and/or hydrophilic monomers is within the range 0.01 to 0.10.

If an additional hydrophobic and/or hydrophilic monomeric component is present, then any suitable hydrophobic and/or hydrophilic monomers may be used. In a particular embodiment of the invention, the hydrophobic and/or hydrophilic monomers are N-substituted acrylate or N-substituted methacrylate monomers. The ester linkages of acrylate or methacrylate monomers are less sensitive to temperature than, for example, the amide groups of the temperature-responsive acrylamide and methacrylamide monomers. Monomeric components comprising allyl ethers can also offer structural and hydrophilic adjustments without interfering with the temperature responsiveness of the co-polymer.

Exemplary hydrophobic monomers include N-alkyl substituted acrylate or N-alkyl substituted methacrylate monomers, such as dodecyl methacrylate, and N,N-di-alkyl substituted acrylates and N,N-di-alkyl substituted methacrylates. The length of the alkyl chains may range from 1 to 20 carbon atoms (and is suitably 1 to 16 carbon atoms). Further exemplary hydrophobic monomers include acrylate and methacrylate monomers substituted with one or more hydrophobic amino acids, such as alanine, valine, leucine or isoleucine.

Exemplary hydrophilic monomers include N-hydroxyalkyl substituted methacrylates or N-hydroxyalkyl substituted acrylates, such as hydroxyethyl acrylate, hydroxyethyl methacrylate or their homologues such as, N-isopropanolmethacrylate or N-isopropanolacrylate. The hydroxyalkyl groups may comprise 1 to 20 carbons, suitably 1 to 16 carbons atoms, and even more suitably 1 to 6 carbon atoms. Further exemplary hydrophilic monomers include acrylate and methacrylate monomers substituted with one or more hydrophilic amino acids, such as serine and lysine.

For both the hydrophilic and hydrophobic monomers, the number of carbon atoms selected in the alkyl side chain, as well as the extent of any branching present in the chain, whether it is mono- or di-substituted, and the presence or absence of any hydrophilic substituent groups (e.g. one or more hydroxy groups), will all affect the overall hydrophobic/hydrophilic character of the resultant co-polymer and ultimately its performance as a substrate coating for cell growth and detachment applications. Accordingly, the selection of the appropriate hydrophobic and/or hydrophilic monomers enables the surface properties of the co-polymer and the resultant polymeric film to be optimized for a particular cell type. A person skilled in the art will be able to select the appropriate type and number of amino acids for the hydrophilic and hydrophobic side group in order to provide the desired properties of the copolymer film formed from the co-polymer. The cross-linking means present in the co-polymers of the invention may be any suitable means for forming a covalent bond between the co-polymer and the substrate surface. In order to covalently bind the co-polymer to the substrate surface it is necessary to provide reactive groups as part of the co-polymer structure that are capable of reacting with functional groups present on the substrate surface to form covalent linkages. A person skilled in the art will be able to select suitable functional groups for this purpose.

Commonly used substrate materials include glass, stainless steel, and treated plastic surfaces. These surfaces possess surface hydroxyl groups which can be utilised to react with functional groups present in the co-polymer structure to form a covalent bond. Other surfaces may possess functional amine groups that can be used to form covalent bonds with the cross-linking means of the co-polymer.

Accordingly, in a particular embodiment, the cross linking means is adapted to react with functional amine and/or hydroxyl groups present on the substrate surface.

Suitably, the cross-linking means additionally forms a covalent bond between the adjacent polymers or between adjacent monomers of the same polymeric chain.

In an embodiment of the invention, the cross-linking means is a functional reactive group present as part of the temperature responsive or hydrophilic/hydrophobic monomeric units.

In further embodiments of the invention, the cross-linking means is an additional monomeric constituent of the co-polymer having a functional group that reacts with functional groups present on the substrate surface. Accordingly, in such embodiments, the invention provides a temperature-responsive co-polymer for forming a film coating on the surface of a cell culture substrate, wherein the co-polymer comprises temperature-responsive hydrophilic monomeric units, hydrophilic and/or hydrophobic monomeric units and a cross-linking monomer component to covalently bind the co-polymer to the substrate surface.

In a particular embodiment of the invention, the cross-linking means is a silane crosslinking group that is incorporated into the co-polymer structure. Particular examples of suitable silane cross-linking groups include methoxysilane groups, such as trimethoxysilane, ethoxysilane groups, such as triethoxysilane, and/or halosilanes, especially chlorosilanes such as trichlororsilane.

In an embodiment, the cross-linking means is a silane cross-linking group selected from a methoxysilane group, such as trimethoxysilane, and/or an ethoxysilane groups, such as triethoxysilane.

In a further embodiment, the crosslinking means is a chlorosilane such as trichlorosliane.

In a further embodiment of the invention, the silane cross-linker is present as a functional group on an additional monomeric component of the co-polymer backbone structure. A particular example of such a monomeric component is 3-trimethoxysilanepropyl methacrylate, 3- triethoxysilanepropyl methacrylate. Any other cross-linking groups or those with different spacer carbon chain lengths to the acrylic or methacrylic double bond will also suffice.

As an alternative, the concept of cross-linking using natural protein composites such as mussel proteins could also be utilised to promote the formation of covalent bonds between substrate and the temperature-sensitive co-polymer film [Lee et al., Adv. Mater. 2008, 20, 1619-1623]. The enhancement of this cross-linking can be ensured by the application of a primer coating of a synthetic co-polymer containing both amine and/or catechol functional groups. Extensive research has demonstrated that these catecholamine molecules are capable of mediating adhesion to most noble metal, organic and inorganic surfaces, mimicking the strong adhesive behaviour of mussel proteins.

The lack of surface functional groups, such as amine and hydroxyl groups, on some substrate surfaces may lead to the detachment of the temperature-responsive film. In such cases, a suitable primer coating could be used to facilitate the formation of covalent bonds between the substrate and the temperature-responsive film. The primer coating is applied to the surface of the substrate material and will comprise a sufficient amount of functional groups, such as amine and/or hydroxyl groups, for the temperature-responsive co-polymer to graft to. Any suitable primer coating may be used.

Thus, many existing cross-linking approaches can be deployed to achieve the present goal of forming a temperature-responsive co-polymer film and this invention does not intend to be bound to any specific approach as far as the effort of promoting the binding of the temperature-responsive film is concerned.

Suitably the cross-linking means is adapted to initiate cross-linking with functional groups present on the substrate surface and within the film by heating or UV radiation. Suitably, the cross-linking occurs during annealing and/or sterilisation of the polymeric film.

The cross-linking means is suitably present in an amount sufficient to form sufficient cross-linkages within the co-polymer film and between the co-polymer film and the substrate surface to form a durable and robust film coating.

In a particular embodiment, the cross-linking means is present on between 0.1 to 50% of the monomeric units present in the polymer. In a further embodiment, the cross-linking means is present in between 0.1 to 10% of the monomeric units present in the polymer. In a further embodiment, the cross-linking means is present on between 0.1 to 5% of the monomeric units present in the polymer.

For cell attachment and growth, amino acids are often the better chemical constituents for surface biocompatibility. Hence, the substituents on the co-polymer side chains or backbone can include amino acids as indicated above. Any suitable amino acids or peptide sequences may be utilised as substituents on the polymer. There are some 20 natural amino acids and many *de novo* homologues, and the choice for the appropriate combination depends on the particular cell culture and operational conditions. Furthermore, cell attachment and growth often requires particular amino acid sequences such as RGD, RGDS or peptide sequences such as growth factors. These short and long peptide sequences and proteins can be linked to the methacrylamide or acrylamide monomers for co-polymerisation. They can also be linked to the preformed film with the help of another cross-linker such as ethylene glycol bis[succinimidylsuccinate] (EGS).

Physical adsorption of polypeptides, extracellular matrix proteins or growth factors onto thermo-responsive co-polymer film surfaces may also lead to the improvement of cell attachment and growth as already demonstrated with other surfaces (Moran et al. J. R. Soc. Interface 2007, 4, 1151-1157; Yaseen et al., Biomaterials 2010, 31, 3781-3792).

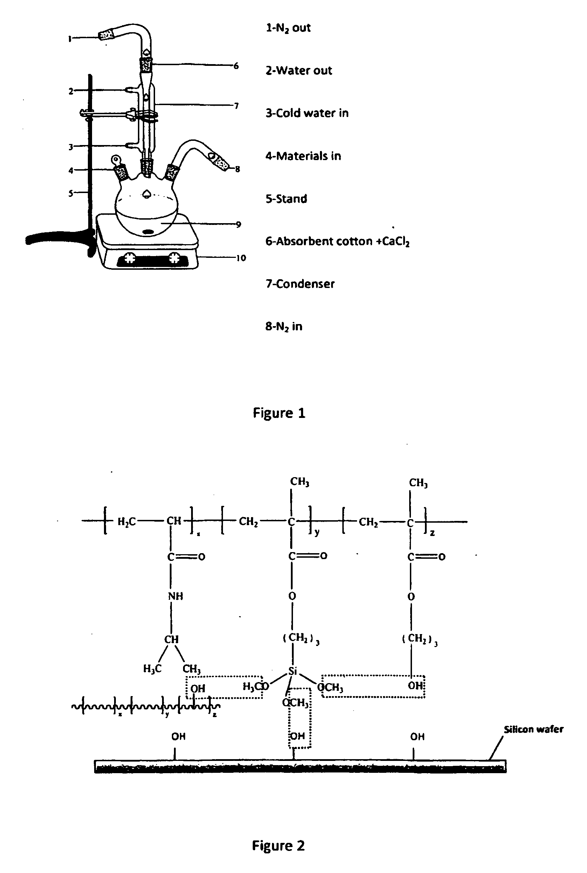

In a particular embodiment of the invention the co-polymer is poly(N-isopropylacrylamide -co- hydroxypropyl methacrylate (HPM) -co- 3-trimethoxysilylpropyl methacrylate (TMSPM)), which is abbreviated to "PNIPAAm co-polymer". The N-isopropylacrylamide is the main monomer and provides the temperature-responsive component of the polymer. The hydroxypropyl methacrylate is a non-temperature-responsive hydrophilic component. The additional hydroxyl groups provided by the hydroxypropyl methacrylate make the resultant films formed from this co-polymer more hydrophilic and also contribute to the softness of the film. The 3-trimethoxysilylpropyl methacrylate provides the cross-linking means. The chemical structures of these monomeric units are shown in Figure 2. In this type of copolymer, a representative set of molar ratios of NIPAAm:HPM:TMSPM is 0.9:0.05:0.05 or a monomer ratio of 20:1:1.

In a further embodiment of the invention, the co-polymer is a PNIPAAm co-polymer as defined above which further comprises, as an additional hydrophobic monomer, such as, for example, dodecylmethacrylate. In an exemplary embodiment, the co-polymer is NIPAAm:HPM:TMSPM:dodecylmethacrylate, optionally with a monomer ratio of 16:1:1:2.

### Synthesis

The present invention provides a process for the preparation of a temperature-responsive co-polymer as defined herein, the process comprising the polymerisation of temperature-responsive monomeric units and one or more additional hydrophobic and/or hydrophilic monomeric units.

Where the co-polymer comprises a cross-linking means which is present as an additional monomeric component of the co-polymer structure, the process will accordingly comprise the polymerisation of temperature-responsive monomers, one or more additional hydrophobic and/or hydrophilic monomers and the cross-linking monomers.

Any suitable polymerisation process known in the art may be utilised to form the co-polymers of the present invention and a person skilled in the art will appreciate that the particular polymerisation process selected will be dictated by the nature of the monomers concerned. A person skilled in the art will also be able to select appropriate polymerisation conditions, including the selection of reaction conditions (e.g. reaction temperatures, reaction times) and the selection of any additional reagents that are required (e.g. catalysts and reaction initiators).

In embodiments of the invention where methacrylic or acrylic monomers are used, the co-polymer can be formed by dissolving the monomers together in a suitable solvent and initiating the polymerisation of the monomers using ultra-violet light or a chemical initiator, such as AIBN (azobisisobutyronitrile). The resultant co-polymer can then be purified and collected by any suitable means, such as, for example, precipitating the co-polymer in the presence of a suitable non-solvent for the co-polymer (such as n-hexane).

Any suitable solvent may be used for the polymerisation reactions defined herein. Particular examples of suitable solvents include methanol, ethanol, n-propanol, iso-propanol and acetone.

In a further aspect, the present invention provides a temperature responsive co-polymer obtainable by any of the processes defined herein.

In a further aspect, the present invention provides a temperature responsive co-polymer obtained by any of the processes defined herein.

### Formation of the temperature-responsive copolymer film

The present invention also provides a method of forming a temperature-responsive film coating on the surface of a substrate, said method comprising dissolving a temperature responsive co-polymer as defined herein in a suitable solvent to form a solution, applying the solution onto the surface of the substrate, removing the solvent and annealing the co-polymer to the substrate surface.

Removing the solvent deposits the co-polymer. The deposited co-polymer is suitably heated to anneal the co-polymer film.

In a particular embodiment, the method further comprises an additional step of sterilising the substrate.

Any suitable means of applying the solution onto the surface of the substrate may be used. For example, the solution may be poured or sprayed onto the substrate surface. Alternatively, techniques such as spin coating or dip coating may also be used.

The solvent may be removed by allowing it to evaporate at ambient temperature or by heating the substrate to cause the solvent to evaporate.

Any suitable solvent capable of dissolving the temperature responsive co-polymer of the present Invention and which is sufficiently volatile to be easily removed from the surface of the substrate by evaporation may be used to form the solution. Particular examples of suitable solvents include methanol, ethanol, propanol and acetone. Any mixed solvents taking into account of the hydrophilic and hydrophobic balance of the co-polymers, a binary mixture of ethanol/hexan or mixture of ethanol/chloroform/hexane, are also suitable.

The co-polymer film may be annealed by heating to a desired temperature. The temperature required will vary depending on the nature of the cross-linking means. For copolymers containing TMSPM cross-linking groups, preferable annealing temperatures range between 50-100 °C and the annealing time ranges from 0.5 to 4 hours, although the precise conditions may depend on type of substrate used, such as plastics (e.g., tissue culture plastic plates and vessels, polystyrenes), glass, stainless steel. Higher annealing temperatures can enhance the degree of cross-linking between co-polymer and the substrate and within the co-polymer film, improving film grafting onto the substrate surface whilst constraining the extent of film expansibility.

In a particular embodiment of the invention, the cross-linking means reacts with functional groups present on the surface of the substrate to covalently bind the co-polymer film to the substrate surface during the annealing and/or sterilisation steps of the process.

In a further aspect, the present invention provides a coating solution for coating the surface of a substrate, the coating solution comprising a temperature-responsive co-polymer as defined herein dissolved in a suitable solvent as defined above.

In a particular embodiment of the invention, the coating solution comprises a temperature responsive co-polymer of the present invention dissolved in ethanol.

In a further aspect, the present invention provides a co-polymer film coating obtainable by any one of the processes defined herein.

In a particular embodiment, the polymeric film may comprise a temperature-responsive co-polymer as defined herein as the sole co-polymer material present in the film.

In an alternative embodiment, the polymeric film may comprise additional polymers such as, for example, amino acids, bioactive peptides or growth factors.

In a further aspect, the present invention provides a kit of parts comprising, as a first component, a temperature responsive co-polymer as defined herein and, as a second component, a suitable solvent for dissolving the co-polymer to form a solution which can subsequently be applied to the substrate surface.

Suitably, the kit of parts further comprises instructions detailing how to use the component parts of the kit.

The component parts of the kit are also preferably sterile so that they can be aseptically applied to the surface of the substrate.

### Properties of the co-polymer film

The polymeric films formed by the co-polymers of the present invention provide a surface upon which cells can grow in culture, and then be dislodged by a reduction in temperature (below the LCST) and harvested. In order to achieve this objective, it is desirable that the co-polymer films are uniform in thickness and their surface properties, and adequately bound to the substrate surface (so as to prevent detachment during use).

It is a further feature of the co-polymer films of the present invention that, at a temperature at or around the incubation temperatures commonly used in cell culture processes, typically at or around 37°C for mammalian cell cultures (i.e. between 35°C and 39°C), the temperature responsive monomeric units of the co-polymer are dehydrated so that the polymeric film presents a hydrophobic surface which the cells can readily adhere to and grow. However, when the temperature is decreased to below the LCST of the copolymer (which for mammalian cell cultures is preferably between 20°C and 35°C), the temperature responsive monomers become hydrated. This hydration causes the co-polymer film to swell and present more hydrophilic surface to the cells adhered to the surface of the film. Without wishing to be bound by any particular theory, it is believed that the swelling of the co-polymer film and the more hydrophilic nature of the surface results in the cells being dislodged from the surface of the substrate.

The thickness of the polymeric film will be dictated by the concentration of the coating solution utilised during co-polymer film formation, as well as lifting speed utilised in dip coating or the spin speed utilised in spin coating procedures. The nature of solvent and coating temperature may also affect film thickness and uniformity. For particular cell types there will be an optimal film thickness and outer surface properties that provide optimal adhesion and detachment, which can be controlled by varying the concentration of the coating solution while keeping all other coating conditions constant. Suitably, the co-polymer films are nanofilms having a thickness in the nanometer range.

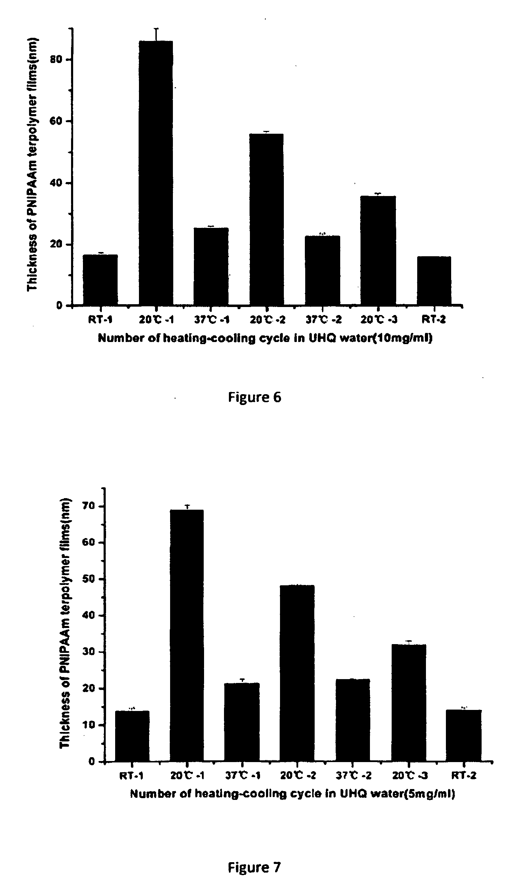

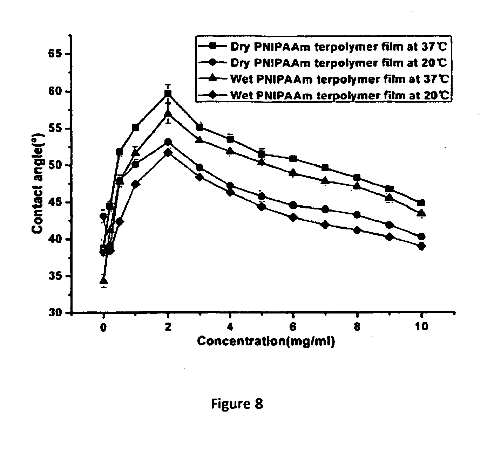

Figures 6 and 7 show that the concentration of coating solution affects the film thickness. Increasing coating solution concentration increases film thickness. The control of film thickness can be critical to achieving effective cell detachment because different cell types require films of differing thicknesses for optimal adherence and detachment. Table 1 in Example 6 shows that HeLa cells are best detached when the particular co-polymer films were about 5-15 nm thick (dry thickness). In contrast, the optimal film thicknesses for detachment of BMMSC cells and HEK293 cells were much thinner. When hydrated, these co-polymer films would expand quite extensively, but the exact extent of expansion is dependent on solution temperature, as evident from Figures 6 and 7 as well. Without being constrained by any particular theory, the optimal thickness range for a particular cell type may vary with the exact composition of the co-polymer and conditions used for film coating and annealing. It is well expected that these conditions are likely to affect the film inner structure and the outer surface properties that would inevitably affect the temperature-response of the film and its interaction with the cell outer surface. Changes in the outer surface properties are marked by variations in surface contact angle. Figure 8 shows examples of variations of surface contact angle with film thickness (controlled by polymer concentration) in dry and wet conditions. In this regard, the need for different film thicknesses by different mammalian cells is clearly reflected by the need for different surface properties. Changes in co-polymer composition and annealing conditions may result in a different set of relation, to achieve similar outer surface properties and film expansibility for adherence and detachment of a given type of cell.

### Substrates for cell culture

In a further aspect, the present invention provides a substrate having a surface upon which cells can grow and divide in culture, wherein said surface is coated with a polymeric film coating formed from a temperature responsive co-polymer as defined herein and said polymeric film coating is covalently bound to the substrate surface.

In a particular embodiment, the substrate is the internal surface of a cell culture vessel which can be used to grow cells in culture medium. Accordingly, this may be any suitable vessel such as, for example, a cell culture flask or plate. Thus, a particular aspect of the present invention provides a cell culture vessel having a substrate surface upon which cells can be grown and propagated in culture media, wherein said surface is coated with a polymeric film coating formed from a temperature responsive co-polymer as defined herein and said polymeric film coating is covalently bound to the surface.

In an alternative embodiment, the substrate is a separate article that can be placed within a cell culture vessel as defined herein. The article may be a solid body which is provided with a coating of the co-polymer of the present invention or, alternatively, it may be porous, for example in the form of a mesh.

Thus, in a further aspect the present invention provides an article configured to be placed within a culture vessel, said article comprising a substrate material coated with polymeric film comprising a temperature responsive co-polymer as defined herein and wherein said polymeric film coating is covalently bound to the surface of the substrate.

The substrate may be formed from any suitable material. Suitably the surface comprises suitable functional groups that can be utilised to form covalent bonds with the cross-linking means present in the co-polymers of the invention. Particular examples of suitable substrates include noble metals, stainless steels, ceramic composites including TiO₂, glasses (SiO₂) and treated plastics and other substrates such as polystyrenes, PMMA, Teflon, organic glasses. For surfaces that are difficult to bind with the temperature-responsive co-polymer film due to either mismatch in hydrophobicity or absence of suitable functional groups (e.g. amine and/or hydroxyl groups), a primer coating can be used to promote adhesion with the substrate as well as presentation of amine and hydroxyl groups for the temperature-responsive co-polymer to anchor.

### Applications

The coated substrates defined herein can be used in cell culture to provide surfaces that cells can adhere to and grow.

Thus, the present invention provides a method of culturing cells comprising seeding cells on a substrate as defined herein in the presence of a culture medium and maintaining the temperature at an incubation temperature to allow the cells to grow and divide, and then reducing the temperature to harvest the cells.

The temperature is suitably reduced below the incubation temperature to dislodge the cells from the substrate. The cells are suitably harvested by withdrawing the culture medium.

Suitably the temperature is reduced to below 35°C (preferably 30°C or below, more preferably 25°C or below). This can be achieved by simply removing the culture from the incubator and allowing it to cool to room temperature. Alternatively, it may be cooled in some other way, for example by refrigeration or the application of cooled culture media. Once the culture has been allowed to cool for a sufficient time, the cells can be harvested by withdrawing the culture medium in which the dislodged cells will be present. The substrate may then be either re-used or discarded.

In a further aspect, the present invention also provides a method of dislodging cells from a substrate surface which is coated with a temperature responsive co-polymer as defined herein, the method comprising cooling the substrate such that the temperature responsive co-polymer hydrates and thereby causes the cells present on the substrate surface to be dislodged.

Cooling the substrate suitably involves cooling the substrate to a temperature below the incubation temperature.

The temperature-responsive co-polymers and substrates of the invention provide a number of advantages. In particular, the coating process is easy to implement and any size and shape of the cell culture substrate or vessel (e.g. flasks, tubes and bioreactors) can be coated. The process can also be easily scaled up to enable mass production.

Using the co-polymer film coated flasks, it has been demonstrated that HaLa cells, HEK293 cells and mouse MSc cells cultured at 37 °C of substrate surfaces provided with a co-polymer coating according to the present invention were able to detach these cells upon decrease in temperature down to 20 °C, showing the effectiveness of the surface coating. Furthermore, the cell culture substrate could be re-used without the need for surface cleaning. This benefit is very attractive for the development of enzyme-free and reusable cell culture flasks and is also effective for coatings of bioreactor vessels and tissue scaffolds such as the inner and outer surfaces of model veins and arteries. The same approaches can also be used to produce 2D films of skin cells, vascular endothelium cells and cardiac myocytes. As microfluidic culturing devices are becoming popular for studying cell growth coupled with the flexibility for delicate control and manipulation of cell-cell interactions, surface coatings using our polymers will allow reliable control of surface chemistry when the devices are reused.

### EXAMPLES

The invention will now be described in more detail in relation to the following illustrative examples.

### Materials

The monomer NIPAAm was purchased from Wingch (China); it was above 98% pure and freshly recrystallised in hexane, followed by freeze-drying before use. The monomers hydroxypropyl methacrylate (HPM) and dodecyl methacrylate (DM) were purchased from Aldrich. The glass coverslips or silicon cross-linking agent 3-trimethoxysilylpropyl methacrylate (TMSPM) was also purchased from Aldrich. The initiator 2, 2-azobisisobutyronitrile (AIBN) was purchased from BDH (UK) and was fully recrystallised in ethanol followed by freeze-drying before use. The solvents including ethanol, acetone and n-hexane were provided by Aldrich.

The water used was processed using Elgastat ultrapure (UHQ) system. The silicon wafers were purchased from Compart Technology Ltd (UK) and were cut into 1×1 cm² cuts before use. They were cleaned by 5% Decon solution, followed by rinsing with UHQ water. The glass coverslips with diameter of 13 mm were purchased from VWR (Belgium). All plastic vessels (except those for single use in cell culture) were cleaned by soaking them in 5% Decon solution. All glassware was immersed into piranha solution (H₂O₂: H₂SO₄ = 1:3 by volume) for 30min, followed by abundantly rinsing with tap water and UHQ water.

Cell culture involved the use of Dulbecco's modified Eagle's medium-low glucose (DMEM-LG), penicillin-streptomycin, ampicillin sodium from Sigma; fetal bovine serum (FBS), trypsln+ethylenediamine tetraacetic acid (EDTA), trypan blue and phosphate buffered saline (PBS) from GIBCO.

### Example 1 - The synthesis of poly (N-isopropylacrylamide-co-hydroxypropyl methacrylate -co- 3-trimethoxysilypropyl methacrylate) (abbr. PNIPAAm co-polymer)

The PNIPAAm co-polymers were synthesised by free radical polymerisation according to reported protocols (Cho et al., Biomaterials, 2004, 25: 5743-5751; Moran et al., J Biomed Mater Res, 2007, 81 A: 870-876; Zhang et al., Biomacromolecules, 2006, 7: 784-791; Cao et al., Journal of Controlled Release, 2007, 120: 186-194) with some modifications.

For a typical synthesis, 2g of NIPAAm, 0.13g (5%mol NIPAAm) of HPM, 0.22g (5%mol NIPAAm) of TMSPM, and 8 ml of absolute alcohol were poured into a three necked flask with a condenser, and subsequently purged with nitrogen for some 10min. 0.0319g (1 %mol of the total (NIPAAm + HPM + TMSPM)) of AIBN was added into the mixture solution. The mixture was first purged with nitrogen flow for about 10 minutes, then heated up and kept under heating and stirring at a constant temperature of 60°C for 12 hours under nitrogen protection. The synthetic set up for the production of PNIPAAm co-polymers is schematically illustrated in Figure 1. After polymerisation, the solvent ethanol was evaporated and a small amount of acetone was then added into the remaining sample to dissolve it. The liquid was then dropwise added in n-hexane for precipitation of the sample. The precipitation process was repeated three times using acetone as solvent and n-hexane as non-solvent. Eventually, the product was dried at -60°C in the vacuum freeze dryer for 24 hours and then stored in a refrigerator for use.

Co-polymers incorporating DM monomer with and without HPM monomer can be synthesized similarly. Incorporation of DM monomer into the co-polymers tends to increase their hydrophobic character. Hydrophobicity can also be improved by replacing HPM monomer with hydroxyalkyl groups with greater alkyl chain lengths. Without being constrained by any theory, increase In DM or similar monomers may help promote layered structuring within the annealed film. This effect thus improves the presentation of NIPAAm and other functional groups such as peptide sequences, polypeptides (growth factors) and proteins (stem cell markers) on the outer film surface because the accessibility to these groups may become important for the adhesion and growth of certain cells without altering their properties (e.g., phenotypes, biological and biochemical signatures). Incorporation of DM-like monomers is also envisaged to make film coating much easier and less sensitive to the exact thickness coated.

### Example 2-Characterisation of PNIPAAm co-polymer

### (i) Fourier Transform Infrared Spectroscopy (FTIR)

The FTIR spectra for the PNIPAAm co-polymers were determined by mixing the co-polymer with potassium bromide (KBr) pellets and then pressing the mixture into a transparent disc. The FTIR spectra were recorded on a Nicolet magna 750 FTIR spectrophotometer in the wave number range of 4000 - 500cm⁻¹.

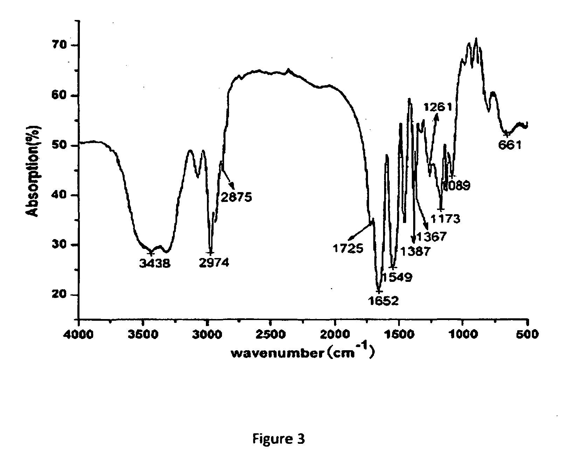

The FTIR spectrum of PNIPAAm co-polymer is displayed in Figure 3. PNIPAAm was characterised by the typical absorption peaks of amide I and amide II at 1652 and 1549cm⁻¹ respectively and the characteristic doublet absorption peaks of isopropyl groups at 1387 and 1367cm⁻¹. The strong broad absorption peaks at 3438cm⁻¹ could be attributed to hydroxyl groups in HPM. Furthermore, the absorption peaks at 2875 and 1089cm⁻¹ proved the presence of siloxane groups. Also, the absorption peaks at 1725, 1261 and 1173cm⁻¹ were assigned to ester groups, which came from HPM and TMSPM.

### (ii) Dynamic Light Scattering (DLS)

DLS was undertaken using a solution of the co-polymer to determine the solubility and the temperature-response of the co-polymer. The concentration for PNIPAAm co-polymer of 1mg/ml was used in all.DLS experiments. The heating and cooling rates influence the measured hydrodynamic diameters; the higher rates tend to give a smaller size. Therefore, the same rate was applied between parallel measurements. In a typical DLS experiment, the PNIPAAm co-polymer was dissolved in UHQ water under stirring at room temperature for 12 hours. The solution was then filtered with a 0.2µm filter to remove any insoluble particles before it was added into the sample glass cuvette. The temperature of the experiments was changed between 15 and 45°C and the temperature fluctuations were kept within ±0.1°C. After each change of temperature, the reading was made at least 10min after the solution had reached the desired temperature and the co-polymer chains had been in the equilibrium state. Each correlation curve was accumulated 30-50 times to eliminate noise and the average values were applied to analyse the trend for the hydrodynamic diameters under different temperatures. The instrument used was a Malvern Nanosizer (Nano-S-ZEN1600, Malvern Instruments Ltd, UK) with a scattering angle fixed at 173 °.

According to the Rayleigh approximation, the intensity of the scattered light is proportional to the 6^{th} power of the particle diameter (d). Thus, particle size, rather than number, is the decisive factor of the intensity distribution. The intensity distribution can be converted into a volume or number distribution by Mie theory. All the DLS results presented herein were derive from volume distribution.

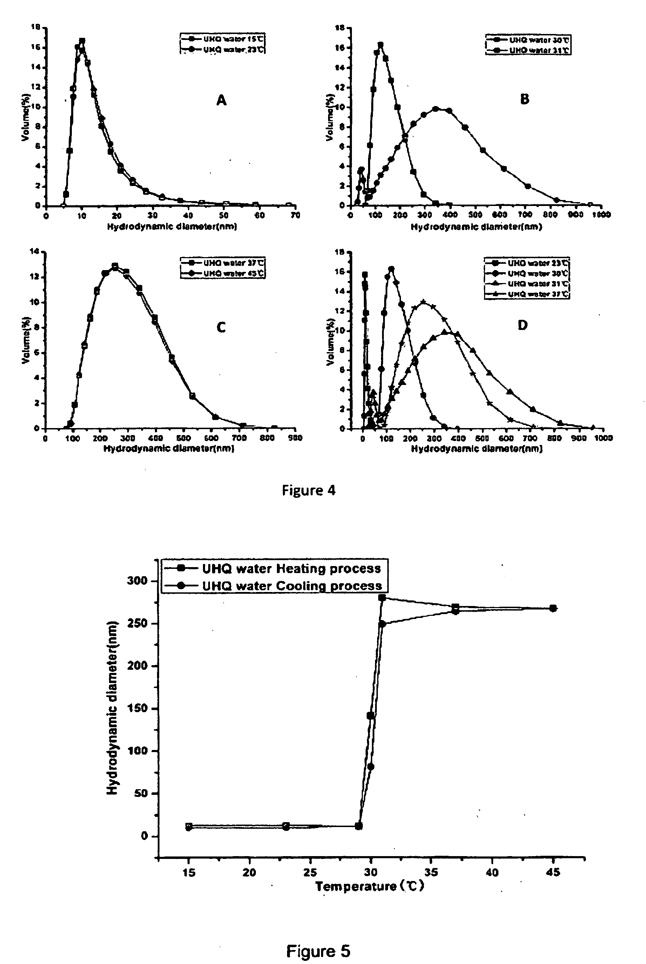

Figure 4 shows representative scattering profiles from the DLS experiments. There are four types of size distributions. Below LCST (Figure 4A), the hydrodynamic diameters of the PNIPAAm co-polymers were distributed from 5.6nm to 58.8nm, with the peak centred at 10.1 nm. The polydispersity index (PDI) width had the values of 6.2nm and 5.9nm at 15°C and 23°C, respectively, indicating that the PNIPAAm co-polymers were well solubilised in water. The small particle size and narrow distribution demonstrated that there was no major formation of aggregates under these conditions. The molecular size and distribution of the PNIPAAm co-polymers did not change much with temperature below LCST. The free molecular state was indicative of the strong hydrophilic outer shell formed by hydrogen bonding between water molecules and amide linkages and hydrogen bonding between water molecules and hydroxyl groups. The transition occurred above 29°C. At 30°C, the maximum peak shifted to 122nm (Figure 4B) that was increased significantly and the PDI width of 48.5nm was wider obviously. This observation suggested that the release of water molecules led to the co-polymer chain dehydration and the increase in particle size reflected the increasing intermolecular hydrophobic interactions between isopropyl groups and hydrogen bonding between amide linkages. At 31°C, two peaks in the vicinity of 43.8nm (accounting for 3.7% volume) and 342nm (accounting for 9.8% volume) had been observed, which showed that most of PNIPAAm co-polymers aggregated intensively to form large aggregates whilst a small part of them also gathered to form small aggregates, resulting in a bimodal distribution. The average hydrodynamic diameter was estimated to be around 280nm. Further increase in temperature led to similar aggregation, with the size distribution becoming narrowed again. The size distribution at 37°C was basically identical to that at 45°C, showing that the PNIPAAm co-polymers undertook a gradual adjustment of molecular conformations under the combined action of hydrogen bonds and hydrophobic interactions. Thus, the co-polymers remained as hydrated molecules below LCST, but above it they formed nanoaggregates with a wide size distribution. The content of water molecules associated with the PNIPAAm co-polymers played a decisive role in the transformation of the macroscopic states.

The temperature-responses of the co-polymers can be better visualised by plotting the peak diameters against temperature change and the results are shown in Figure 5. At the temperature below 30°C, the diameter of the scattering objects was around 10nm, indicating the fully hydrated co-polymer molecules present in the solution. Heating of the solution led to the start of the structural collapse and the dehydration above 29°C. In the transition range of 29-31 °C, the mean diameters increased about 20 times owing to the formation of intermolecular aggregates with the hydrophobic interactions between the isopropyl groups and hydrogen bonding between amide linkages, implying that the incorporation of additional monomers has decreased the LCST by 2-3°C. Above 31°C, the average hydrodynamic diameters decreased slightly due to changes in hydrogen bonding and hydrophobic interaction. In the cooling process, the PNIPAAm intermolecular aggregates could dissociate into single chain free co-polymers as indicated by the almost overlapping profiles measured. The behaviour of the PNIPAAm co-polymer nanoaggregates in the cooling process also agreed well with that observed in the heating process, however, the average hydrodynamic diameters in the cool-down stage were smaller than those in the heat-up stage at the same temperature, inferring that the transitions of PNIPAAm co-polymer nanoparticles were revertible but not entirely.

### Example 3 - Preparation of PNIPAAm co-polymer films

The PNIPAAm co-polymer at concentrations of 0.2, 0.5 and 1-10mg/ml was dissolved in absolute ethanol with stirring at room temperature for above 12 hours. The solutions were then filtered through a 0.2µm filter membrane to remove any insoluble particles. The PNIPAAm co-polymer films were prepared by spin coating onto glass coverslips(as previously described in Dewi et al., Materials Science-Poland, 2007, 25: 657-662; Critchley et al., J. MATER. CHEM., 1992, 2:157-159; and Prelipceanul et al., Organic thermally stable materials for optoelectronics devices - a linear spectroscopy study [J]. Abstract Book of ICPAM07 Conference lasi, Romania: 1-7) with a single wafer spin processor (WS-400B-6NPP/LITE/AS/8K, Laurell Technologies Corporation, North Wales). The co-polymer films with different thicknesses were formed under the same rotation speed of 3000 rpm and the same spin coating time of 20s but the different concentrations of coating solution (0.2, 0.5 and 1-10mg/ml). The coated films were dried in air for at least 30 minutes under room temperature and then annealed for 3h at 125°C in a vacuum oven to facilitate the reaction of 3-trimethoxysilyl groups present in the PNIPAAm co-polymer with hydroxyl groups present on the surface of the silicon wafers or glass coverslips, and to remove the residual solvent. Finally, the samples were cooled down to room temperature under vacuum. They were removed from the vacuum oven and any unreacted monomers and unconnected co-polymers were extracted by soaking and washing the wafers or coverslips in ethanol and water thoroughly. Eventually, they were washed repeatedly by UHQ water and dried at room temperature in a vacuum oven until constant weight and were then stored in the vacuum desiccators for use.

The ethanol was used as the solvent in this example because of its volatility. Under the same spin coating rotational speed and the same spin coating time, the different solution concentrations were chosen to change the surface properties of PNIPAAm co-polymer films. During the annealing process, solvent removal and anhydrous cross-linking of silanes occurred simultaneously. Figure 2 shows that there were three possible types of reactions of methanol removal while annealed: siloxane groups and hydroxyl groups in the substrate, siloxane groups and hydroxyl groups in self-molecular chain, as well as siloxane groups and hydroxyl groups in other molecular chains. Under the synergistic effect of the three reactions, the PNIPAAm co-polymer films changed from loose/unconnected to compact/cross-linked. Owing to the existence of covalent bonds between film and substrate and the formation of macromolecular network film, the co-polymer films did not fall off from the substrate and the thicknesses of films were relatively constant after long time immersion in water. Many organic/polymer substrates have residual hydroxyl groups that will be sufficient to form crosslinks with methoxysilane groups to immobilise the film against detaching. Titanium and silicon materials or their related composites also bear oxide thin layer on the surface just like glasses. These substrates can also be easily grafted with a stable film coating.

### Example 4 - Characterisation of PNIPAAm co-polymer films

### (i) Determination of PNIPAAm co-polymer film thickness

For the accuracy and consistency of measurements, the thicknesses of the oxide layers on clean silicon wafers were determined with variable angle spectroscopic ellipsometry (Jobin-Yvon UVISEL, France) prior to film coating. The measurement processes were performed following the protocols reported previously by Zhang et al. (Biomacromolecules, 2006, 7: 784-791). Briefly, the beam with the wavelengths between 350-700nm was directed to the sample surface at an incidence angle of about 70° and accurate measurements of the two optical parameters Δ and ψ as a function of wavelength were acquired. The measurement processes were first carried out in air and then in water and consistent oxide layer thicknesses were observed within ±1Å. Likewise, the dry and wet film thicknesses after film coating were also made assuming that the oxide layer remained unchanged. A specially constructed liquid cell was used to facilitate the measurements at the solid/water interface. A pair of fused quartz plates (0.2mm thick) was used as windows and the incoming and exiting beams were aligned perpendicular to the windows. Changes in film thickness as a function of temperature in water were recorded using temperature controlled device (as described in Murphy et al., Biomaterials, 1999, 20: 1501-1511; Tang et al., Macromolecules, 2001, 34: 8768-8776). The solutions inside the cells were heated up to 37°C and cooled down to 20°C and the process was recycled and repeated to examine the extent of film reversibility in its expansion and contraction. The experimental data were analysed using DeltaPsi II software provided by Jobin-Yvon.

### Results and discussion

Spectroscopic ellipsometry was used to determine the thickness of the PNIPAAm co-polymer films over the wavelength between 300nm and 700nm. Before the film coating the measurements were made to determine the precise thickness of the oxide layer on each wafer surface. The oxide layer thickness was then taken to be fixed when the co-polymer film was analyzed. It was found that the refractive index of the dry co-polymer was about 1.5 based on the fitting to a thick film where it was sufficiently sensitive to separate the thickness from refractive index.

Film thickness measurements were made to the layers coated over the co-polymer concentrations ranging from 0.2 to 10mg/ml so that each concentration had a matching film thickness. All the measurements were first made in air, followed by thickness determination in water at 20 and 37°C respectively, and finally were done in air after drying these samples. To check the reversibility, the temperature was cycled 2-3 times. Figure 6 shows an example of thickness changes measured from the film coated at 10mg/ml. The thickness of the dry film was 16.5nm at 20°C. Under water at 20°C, the average thickness of the layer expanded to 85.9nm. This was then dropped to 56.0nm in the second round and further dropped to 35.7nm for the third round. When the temperature was heated to 37°C, the film thickness went down from 25.4nm to 22.7nm, showing much smaller variation. The thickness of the dry film was 15.8nm at 20°C in air after drying it in a vacuum oven following its temperature cycling in water, displaying the consistency with the result of dry film in the first time.

These results showed that upon the first immersion at 20°C, water rushed massively into the co-polymer film possibly due to its open structural network. These water molecules must bind with amide groups and hydroxyl groups through hydrogen bonding, causing the largest film thickness expansion. Upon heating to 37°C, hydrogen bonds between water molecules and amide linkages were partly transformed into hydrogen bonds between the amide linkages, meanwhile, hydrophobic interactions between the isopropyl groups played a dominant role. When the temperature was brought back to 20°C for the second time, the thickness of the co-polymer film dropped to about 65% of the value in the first round. Although the interaction between the hydrophobic groups was not the main factor for swelling, it was responsible for the lack of swelling at 20°C because the hydrophobic stacking must have caused restriction to the extent of expansion as expected from water molecules and amide linkages, causing a substantial reduction in the swelling degree of the co-polymer film. Thus, whilst the main trend of the temperature-responsive changes is the same as observed from hydrodynamic diameters obtained from DLS (data not shown), the difference lies in the steady decrease in the film thickness at 20°C. But the thickness decline did not imply a gradual lose of the co-polymer in the film due to possible partial dissolution because the thickness at 37°C did tend to an almost constant value, confirming that the mass in the film remained intact. Similar results were obtained in the two measurements of dry films before and after immersion in water for several cycles of variable temperature, fully proving the stability of the grafted PNIPAAm co-polymer films.

The temperature-response of the film coated under 5mg/ml of the co-polymer solution was examined similarly. An almost identical trend was observed, that was, the film experienced a large thickness increase at 20°C after the first round of temperature cycle, but further cycles led to an almost constant thickness at 20°C. In contrast, the film thickness at 37°C changed much less. It was however clear that the difference in the thickness between 20°C and 37°C still remained substantial, as can be clearly seen from Figure 7. As will be seen later, such changes in thickness were sufficient to detach cells.

### (ii) Contact angles

The water contact angles of dry and wet PNIPAAm co-polymer films were determined by the sessile drop method at both 20°C and 37°C and the MB-ruler image analysis software was used to estimate the contact angles: The dry films referred to the ones dried under vacuum before measurement. The wet films were obtained by soaking the samples into UHQ water at room temperature. The experimental results were averaged from six repeated runs with standard deviation.

### Results and discussion:

Surface contact angles of the coated films may offer a useful indication of changes in surface wettability and chemical composition. Figure 8 shows variation of the contact angles of the PNIPAAm co-polymer films under dry and wet conditions. It can be concluded that the contact angles of the dry and wet PNIPAAm co-polymer films at 37°C were more than those at 20°C, demonstrating that these films did possess the temperature responsiveness. At each temperature, dry films had greater contact angles than the wet ones, clearly showing the association of water into the wet films. Under a given temperature and dry or wet condition, the contact angles initially increased with solution concentration, reached a maximum, then decreased. The initial contact angle increase must be related to the increased surface coverage of the film, but after reaching the peak value, the steady decrease indicated that the surface hydrophilicity improved gradually with increasing amide and hydroxyl linkages. This observation is important, as different cell attachment and growth may require different surface chemical composition such as the extent of amide bonds. The outer surface chemical properties can therefore be adjusted by adjusting the film thickness.

### (iii) Atomic Force Microscopy (AFM)

The AFM images of dry PNIPAAm co-polymer films grafted on the silicon wafers were acquired from a Nanoscope IIIa system (Digital Instruments, Santa Barbara, CA) in the tapping mode under laboratory conditions. Both topography and phase images of different scan sizes from 500nm to 10µm were obtained simultaneously using a resonance frequency of less than 270 kHz for the oscillation of silicon nitride probes (Olympus) with tip radii of approximately 20nm at a scan rate of 2.959Hz. The most representative AFM images for each sample were selected from more than three times measurements in diverse scan sizes at different surface points. The surface property parameters like root-mean-square roughness (Rq) and height and so on were analysed with Nanoscope III software and were showed using averaged results.

### Results and discussion:

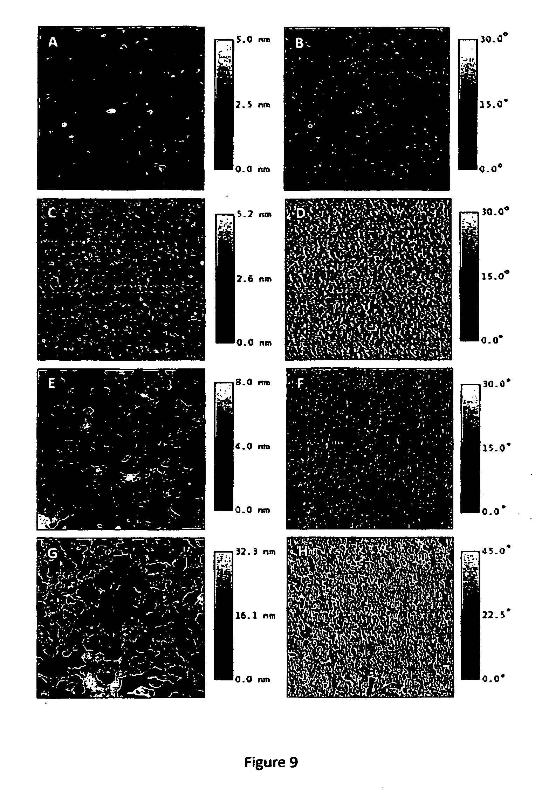

The topography and phase images of PNIPAAm co-polymer films formed by different concentrations are displayed in Figure 9. On the whole, the surface topography varied with the co-polymer concentration, concretely representing in number, height and distribution of peaks. It could be observed that the film surface of 0.2mg/ml was very smooth (Rq ≈ 0.2nm) with some little light dots (height = 2.5-5nm) in Figure 9A, indicating that only a few co-polymers could be deposited on the surface in extremely dilute concentration. As the concentration increased to 1 mg/ml (Figure 9C), the number of distinct peaks increased significantly as a result of that more spots were present and uniformly distributed whereas the average height of peaks were similar to that of 0.2mg/ml, showing that PNIPAAm co-polymer particles grew to cover the silicon wafer gradually and meanwhile connected with each other at the same layer and bind with the substrate during the preparation process. As shown in Figure 9E, topographic image of the co-polymer film with the concentration of 5mg/ml demonstrated a non-uniform surface (Rq ≈ 1.4nm) characterised by the fact that some regions were covered with co-polymer aggregates with peak height between 4 and 8nm, inferring that PNIPAAm co-polymer particles within multilayer were cross-linked to form thicker deposition in higher concentration, whilst, the other regions on the bare silicon wafer surface were exposed to the AFM probes. The co-polymer film of 10mg/ml (Figure 9G) was more inhomogeneous and rougher layer (Rq ≈ 7.0nm, height = 16-32nm) with a non-uniform distribution of co-polymer aggregates, showing an obvious mountain-valley model. The extent of aggregation and cross-linking of co-polymer particles in other words the thickness of co-polymer films increased with increasing concentration. This tendency was well agreed with the results obtained by Ellipsometry.

### Example 5 - Culture of HeLa cells on PNIPAAm co-polymer films

Glass coverslips with PNIPAAm co-polymer films of different surface properties were sterilised for 1 h by UV and then transferred into 24 well tissue culture plates for subsequent use. Some of the glass coverslips were half coated so that the bare glass surfaces worked as control. Before starting cell culture, the coverslips were rinsed repeatedly with PBS and DMEM-LG. HeLa cells were planted on the coverslips immersed in DMEM-LG supplemented by 10% FBS, penicillin-streptomycin and ampicillin at the density of 5.0×10⁴cells/ml and cultured for 96h at 37°C in humid air with 5% CO₂. The growth status of the cells was observed by inverted phase contrast microscope (TE2000-U, Nikon). After aspiration of outspent medium, the cold fresh culture medium (less than 20°C) was introduced, accompanied by gently pipetting. Cell morphology investigation with microscope and adhesive cell number counting with hematocytometer were adopted. The assessments focused on cell growth under culture condition at 37°C and the extent of detachment at 20°C. Those showing healthy growth and swift and clean detachment were considered as the optimal films for HeLa cells.

### Results and discussion:

An interesting observation was that there was no difference between the glass control and the co-polymer coated glass with regard to the cells ability to attach, adhere, move and grow on the coverslip. This indicates that the PNIPAAm co-polymer films had similar biocompatibility to the glass surface.

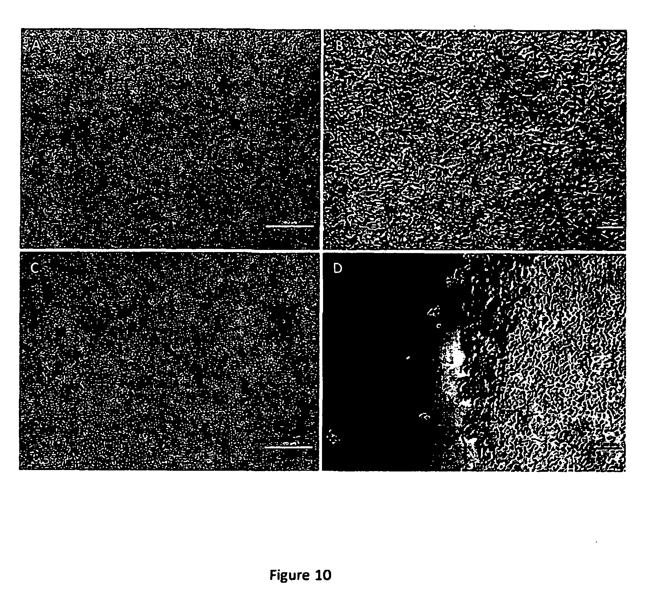

As the film thickness was increased, it became more difficult for cells to attach to the co-polymer coated surface, showing that as film thickness increased, the outer surface of the film had different properties. Thick co-polymer coating surfaces were disliked by the cells. Microscope images of two representative examples of cell adhesion and detachment before and after lowering the temperature are shown in Figure 10. There was no obvious cell growth boundary in the 1 mg/ml group, no matter how the HeLa cells were cultured at 37°C or cooled down to 20°C for 1 h, suggesting that cells seeded on the temperature- responsive film part could adhere and proliferate similarly to the control part above LCST, but cells did not detach from the whole coverslip when the temperature was lowered below the LCST. HeLa cells on the film coated at 5mg/ml adhered and grew well at 37°C and almost completely detached and lifted off from the co-polymer surface within several minutes after cooling down to 20°C, leaving a clear cell boundary when observed under inverted phase contrast microscope. However, at 10mg/ml the film became thicker and most of HeLa cells grew and proliferated on the control glass coverslip while very few cells adhered on the temperature-responsive part of the surface at 37°C. Thus a clearly visible cell boundary occurred at 37°C, due to the dislike of the cells to the film surface. An experimental phenomenon worthy to be pointed out was that the cell boundary after cell detachment was clearly different from that appeared at the beginning, the former often presented irregular wrinkling morphology whereas the latter showed smooth morphology.

It can thus be concluded from the above results that film properties like film thickness, surface wettability and topography affect cell attachment as well as detachment. For HeLa cells there is an intermediate range that is optimal for both cell adhesion and detachment. This observation shows that the control of surface chemistry is important to cell growth and release. A balance of film properties and other coating conditions can lead to effective surface control of cell growth and removal. The phenomenon is expected because the co-polymer contains different functional groups, so changes in film properties can lead to different structure and composition within the film and on the outer surface.

The changes in outer surface properties caused by film thickness are well exhibited from contact angle variation data shown in Figure 8. It can be seen from Figure 8 that as film thickness increases, the contact angle goes down, indicating an increasing surface hydrophilicity. The surfaces of the intermediate film thickness have contact angles of some 50-53°at 37°C and 44-47° at 20°C when it is hydrated. Higher or lower values appear not to offer optimal performance. For the co-polymer films used in this example, it appears that films need to be sufficiently thick to provide enough expulsion. However, too thick a film is no good any longer for cell attachment due to its high surface hydrophilicity.

### Example 6 - Culture of HEK293 cells, BMMSCs and HCASMCs on PNIPAAm co-polymer films

As in Example 5 (HeLa cells), HEK293 cells (human cell line), BMMSCs (mouse stem cells) and HCASMCs (human vascular muscle cells) were also cultured on the glass coverslips coated with PNIPAAm copolymer films that had been sterilized for 1 h by UV and then transferred into 24 well tissue culture plates for subsequent use. Before starting cell culture, the coverslips were rinsed repeatedly with PBS and DMEM-LG. The cells were planted on the coverslips immersed in DMEM-LG supplemented by 10% FBS, 100U/ml penicillin-streptomycin and 50µg/ml ampicillin at the density of 5.0×10⁴cells/ml and cultured for 96h at 37°C in humid air with 5% CO₂. The growth status of the cells was observed by inverted phase contrast microscope. After aspiration of outspent medium, the cold fresh culture medium (less than 20°C) was introduced accompanied by gently pipetting (5-10mins). Cell morphology investigation with microscope and adhesive cell number counting with hematocytometer were adopted.

The results are compared with HeLa cells (Example 5) Table 1.

**Table1. The adhesion and detachment of HeLa cells, HEK293 cells, BMMSCs and HCASMCs on different PNIPAAm co-polymer films**

| Concentration mg/ml) | HeLa cells | | HEK293 cells | | BMMSCs | | HCASMCs | |
|---|---|---|---|---|---|---|---|---|
| | Ad | De | Ad | De | Ad | De | Ad | De |
| 0.2 | √ | × | √ | √ | √ | √ | N/A | N/A |
| 0.5 | √ | × | √ | √ | √ | √ | N/A | N/A |
| 1 | √ | × | √ | √ | √ | √ | √ | √ |
| 2 | √ | × | √ | √ | √ | √ | √ | √ |
| 3 | √ | × | √ | √ | × | N/A | × | N/A |
| 4 | √ | √ | × | N/A | × | N/A | × | N/A |
| 5 | √ | √ | × | N/A | × | N/A | × | N/A |
| 6 | √ | √ | × | N/A | × | N/A | × | N/A |
| 7 | √ | √ | × | N/A | × | N/A | × | N/A |
| 8 | × | N/A | × | N/A | × | N/A | × | N/A |
| 9 | × | N/A | × | N/A | × | N/A | × | N/A |
| 10 | × | N/A | × | N/A | × | N/A | × | N/A |

It can be seen from Table 1 that whilst the appropriate film thickness range (matching the co-polymer concentration between 0.2 and 7 mg/ml) is required for HeLa cells to adhere and proliferate, the other three cells required much thinner film thicknesses matching the co-polymer concentrations less than or equal to 3 mg/ml. The results suggested that whilst HeLa cells require less strict surface growth environments, HEK293 cells, BMMSCs and HCASMCs are stricter. The results indicate that different cells require different outer surface properties for growth and detachment.

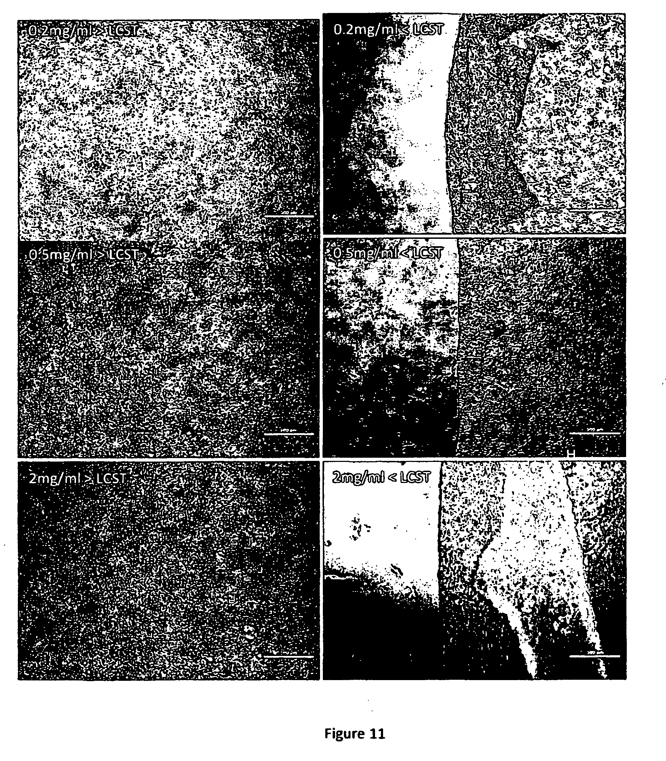

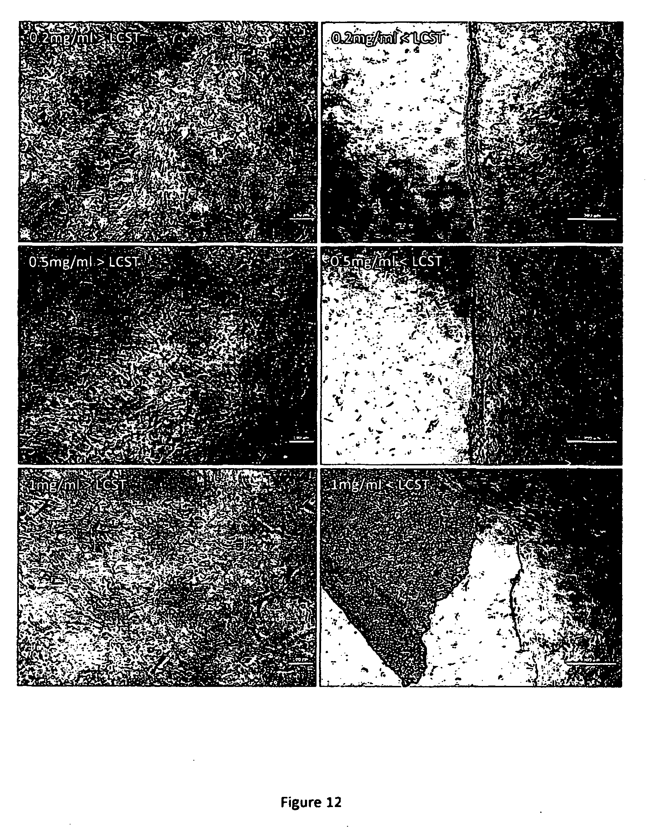

After the temperature was cooled down to 20°C, the cold fresh culture medium was used to replace the warm one in all cases. The HeLa cells could release from the grafted surfaces matching the co-polymer concentrations of 4-7mg/ml. For thinner films coated at 1-3 mg/ml, it appeared to be difficult for the cells to detach. The three other cells could detach spontaneously from all films coated from co-polymer solutions with concentrations less than or equal to 3mg/ml. These results show that delicate balance must be achieved for the strength of cell binding with film surface: cells have to attach to achieve healthy growth, but too strong attachment may not allow cells to be removed through the manipulation of film temperature-responses. Further examples for cell attachment and detachment of HEK293 and MSC cells are shown in Figures 11 and 12.

BMMSCs could attach and grow well at 37°C and fully detach from the PNIPAAm co-polymer films within a few minutes on films coated at 0.2, 0.5 and 1 mg/ml after temperature dropped below 20°C. For each cell type, the cell layer detachment left clear boundary with the control glass side, but the starting location and direction of cell detachment varied. In most cases, the detachment started from the PNIPAAm co-polymer film region and the boundary then moved to the control glass coverslip side.

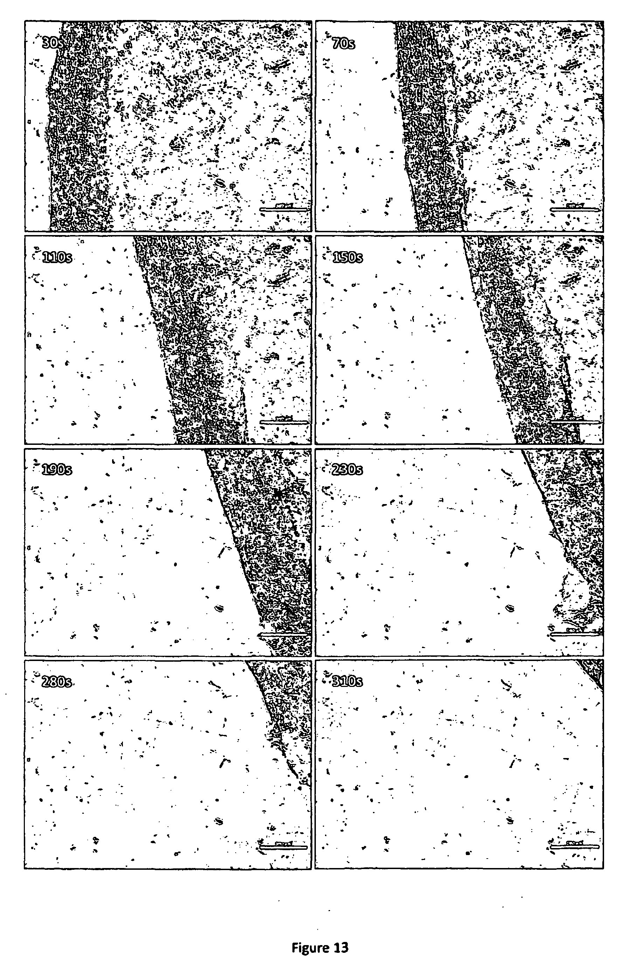

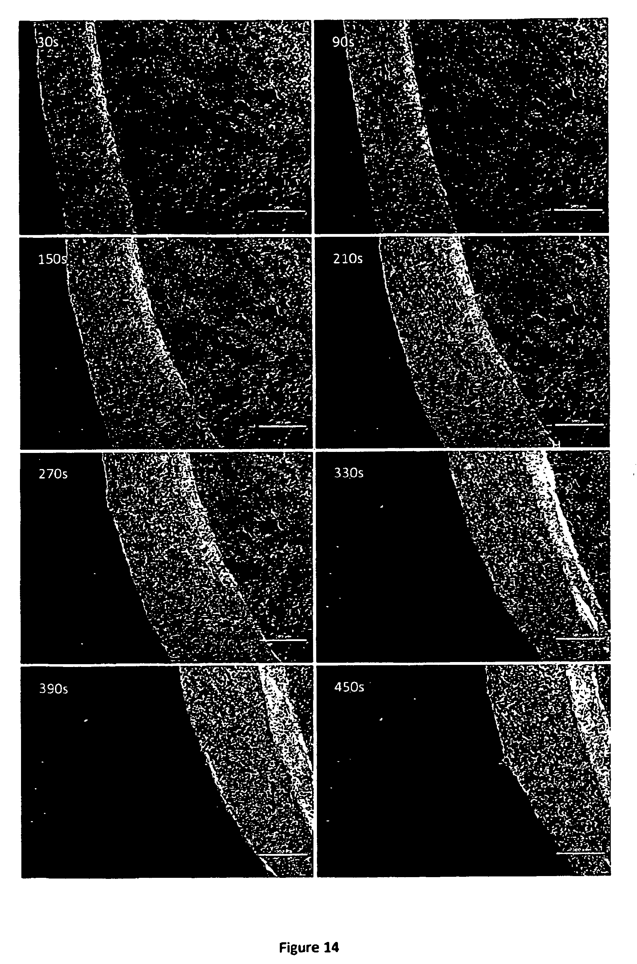

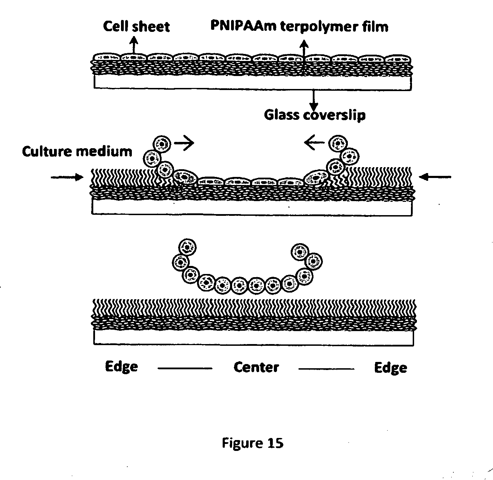

Figures 13 and 14 show the whole processes of cell detachment for BMMSCs and HEK293 recorded. The PNIPAAm co-polymer film surfaces were completely covered with a cell sheet at the starting point. The edge of the film was first exposed to cold fresh culture medium after medium exchange. Accordingly, the film at the cold spot first started to grow thicker and become more hydrophilic, resulting in the blistering of the cell sheet. As the film expansion spread out, the cell sheet on the edge turned round and then released, providing space for culture medium to enter the space between the cell sheet and the film surface, resulting in continuous cell detachment. The cell sheet structuring was found to be more effective to rapid cell detachment than individual cells. Because cells were not only subject to the driving forces caused by the change of film expansion and the transition of surface wettability, but also were pulled by the released cell segments, a dynamic process that is schematically shown in Figure 15.

### Example 7 - Re-usability of PNIPAAm co-polymer films

Previous studies by Canavan et al. (J Biomed Mater Res 75A: 1-13, 2005) and others have indicated that low temperature detachment of cells from the substrate surface is less damaging than mechanical removal and enzymatic digestion. Both mechanical dissociation and enzymatic cleavage change morphological appearances of cells due to damages to extracellular membrane (ECM) proteins. These damages often change cell's activities. In contrast, by culturing cells on temperature-responsive PNIPAAm surface, it is possible to let ECM surface to recover if modest temperature dropping conditions are selected. XPS results of Canavan *et al,* revealed that the loss of the ECM proteins arose from their adhesion to the temperature-responsive surfaces after the cells were lifted off. They also showed that cells grown on surfaces from which an initial layer of cells was removed better promoted cell adhesion, indicating the benefits of residual ECM proteins. Their results were consistent with the observations of improved cell attachment and growth by pre-adsorbing ECM proteins onto surfaces by Moran et al and ourselves. The aim of this study was to illustrate that our co-polymer films can be used for repeated cell cultures without any sign of adverse influence to cell adhesion, growth and detachment.

During the 1^{st} cycle of cell culture, the same steps, conditions and procedures as described in Example 5 were used for treating the coated coverslips, cell planting, culturing and dislodging. After the completion of the 1^{st} cycle, the culturing flask and the coverslips were rinsed carefully with phosphate buffer before they were reused in the 2^{nd} cycle. The same washing step was then taken before the 3^{rd} cycle or more cell culturing cycles was followed. The process was repeated for as long as the coated glass coverslip and flask were reused.

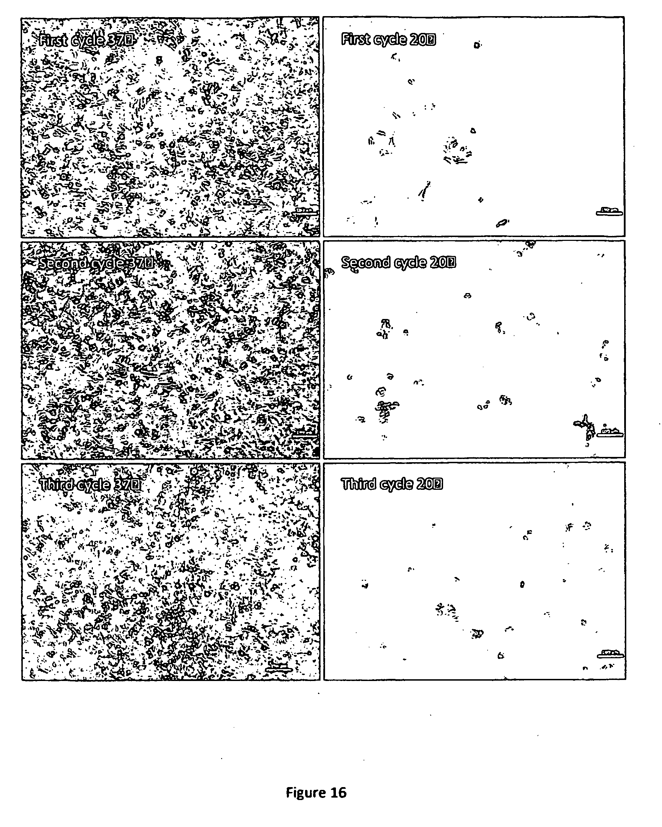

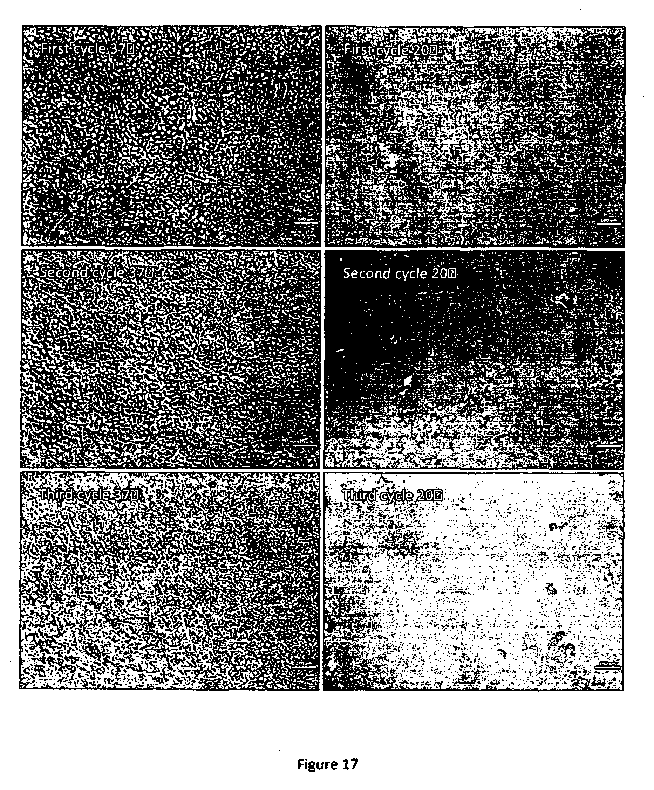

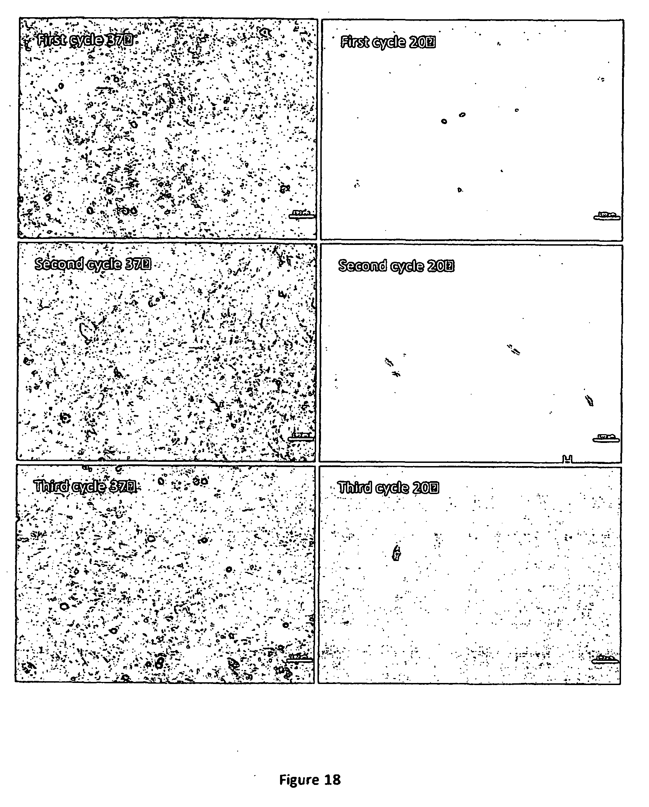

The effect of repeated cell growth and detachment cycles are shown in Figures 16, 17 and 18. The results indicate that the PNIPAAm co-polymer films could be reused for cell adhesion and detachment. The film properties including surface wettability and film thickness were not affected by the processes of long time immersion and successive heating-cooling cycles, thus the used or treated PNIPAAm co-polymer films still provided enough driving force for cell detachment.

It should be noted that for reused films the accumulation of proteins such as fibronectin, laminin and collagen could promote cell growth. The amount of special ECM proteins could increase during the second and subsequent cycles and could then reach equilibration. The accumulation of ECM proteins on the coverslip could provide a friendlier surface environment for reducing the unnatural influence from the surface.

### Example 8 - Culture of (i) human cardiac myocytes and (ii) 3T3 cells on PNIPAAm copolymer films

### (i) Human cardiac myocytes (HCM)

The thermo-responsive PNIPAAm copolymer films were coated onto glass coverslips, as previously described in Example 3, at a concentration of 1 mg/ml. These coated coverslips were placed into wells of a 24 well plate alone. The following controls were included: including bare tissue culture plastic surfaces (24 well plates, TPCS); bare glass coverslip (G); coverslip adsorbed with polylysine (G+L); G+L surface adsorbed with CM medium protein (G+L+CM). To each well 10⁴ cells were seeded.

MTT assays were undertaken to compare the viability of cells grown on each surface against time. MTT assays are widely used to determine the cytotoxicity of potential medicinal agents and other toxic materials, as these agents could result in cell toxicity and therefore metabolic dysfunction and decreased performance in the assay. In this work the performance of the cells grown on co-polymer coated surfaces are compared with different surface controls. Mechanistically, live cells produce enzymes (mitochondrial reductases) that reduce yellow MTT (3-(4,5-dimethylthiazol-2-yl)-2,5-diphenyltetrazolium bromide, a tetrazole) to purple formazan. Thus, this enzymatic activity measures the total activities of the cells (numbers and viability) and is indicative of the extent of active cell growth. In a typical MTT assay using 96 well plates, MTT solution at 5 mg/ml is prepared. To each of the wells 200µl cell culture suspension is added. 200µl cell culture medium is also added in separate wells as blank control. Cells are then allowed to attach to well surface or sample material. To each well 20µl MTT reagent is then added, followed by incubation at 37°C for 3 hours. The MTT/cell culture media is gently removed with a plastic Pasteur pipette. 220µl acidified iso-propyl alcohol is then added and incubated for 30 minutes at 37°C, and the sample is shaken gently every couple of minutes. It is then transferred to another set of wells and the spectrophotometric absorption is measured at 540 and 620 nm. When the assays are undertaken in larger wells, appropriate scale up is required. This will be determined by the surface area of the well used, or of the material in the well.

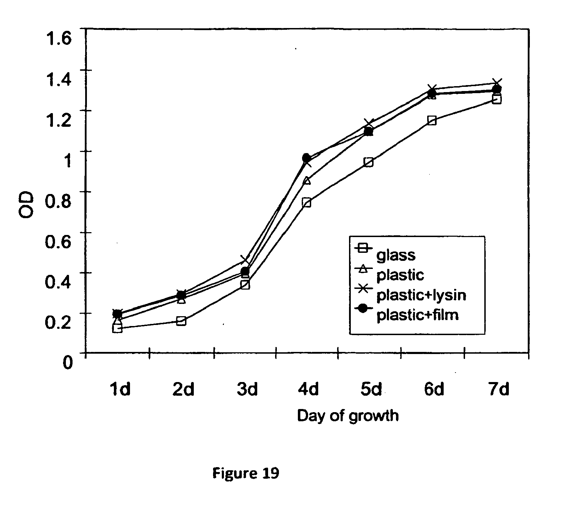

As shown in Figure 19, within experimental errors, there was little difference between the thermo-responsive film, culture plastic and PL (polylysine) adsorbed surfaces. The readings on the glass surface appear to be slightly lower, consistent with the lower cell numbers, but the trend is identical between different surfaces studied. The OD values as shown in the Y- axis of Figure 19 is proportional to the number and viability of cells grown on each surface

Cell adhesion was assessed by washing the loosely attached cells with buffer after 24 hr culturing. The percentages of cells attached to thermo-responsive surfaces with and without polylysine adsorption were between 80 and 83%; those on the bare TPCS was just about 80% and those on the bare glass substrate were between 78 and 80%. Cell morphological observations indicated that after 2 days of culturing, there were little visual differences between cells grown on different surfaces, except that on G+L+CM surface, cell numbers appeared to be greater. GFP transfection again showed no visible effects arising from surface coating on the shape or morphology of the cells.

Hoechst 33258, a specific DNA dye that binds the A-T bonds only, can reveal nuclear fragments indicating apoptosis. Under fluorescence microscope, live cells show smooth, weak but visible light; dead cells do not show any colour. But when cells enter apoptosis stage, cell nuclei and their cytoplasm show stains, usually in the form of small lumps and abnormal nuclear shape. If there are 3 or more fragments or lamps, the cell is regarded as in apoptotic process. No indication of cell apoptosis was noticed from the PNIPAAm coated surfaces.

These analyses thus concluded that the thermo-responsive coated film surfaces did not cause any adverse effects on cell viability and phenotype.

HCM films were also found to easily come off by leaving the culture plates on lab desk to cool down to ambient temperature, placing the plates in 4° C fridge or adding cold cell culture. The cells were dislodged within a few minutes to 30 minutes. It was found that gently pipetting the culture liquid against the cell film surface could speed up the detachment process. Free cell films can be cut and transported to different surfaces.

### (ii) 3T3 cell cultures

This cell culture experiment comprised two parts:
(a) MTT assays as described above have also been used to assess 3T3 cell viability versus time and compare the thermo-responsive film surfaces (coated at 1 and 2 mg/ml ethanol solutions using the main copolymer) with controls (tissue culture plastics (TCPS), bare glass coverslip and polylysine coated surface).
(b) Thermal detachment was again observed by cooling from 37 °C to 20 °C in 15-20 min.

As for (i) above, all cell growth and thermo-responsive cell detachment studies were based on 24-well plates, each sample had 4 repeats. The copolymer films were coated onto coverslips using spin coating, as described in Example 3.

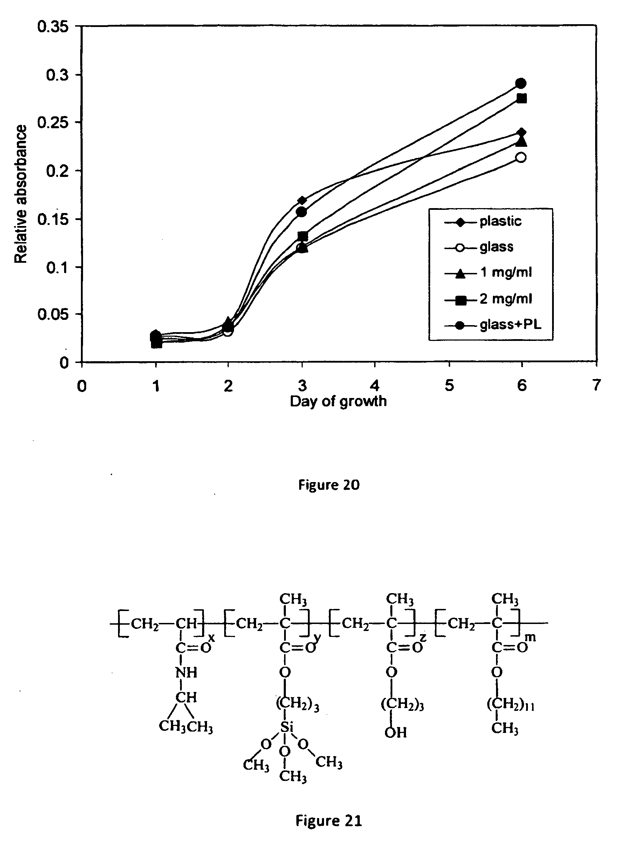

The MTT results are shown in Figure 20 (plotted in terms of relative light absorbance proportional to the number and viability of the cells). Overall, the results show a broadly consistent trend of increase in cell number against time. The data were rather close and showed little scattering over the first two days, but after the third day, the extent of variation became greater. At day 6, the last day of the MTT measurements, cell numbers appeared to be the lowest on glass, but showed the highest on the bare plastic TPCS. Cell numbers from themo-responsive film coated surfaces were intermediate. Given the variations were broadly within the experimental errors, the results again showed little adverse effect arising from the thermo-responsive polymer coated film surfaces.

The thermo-responsive cell detachment of 3T3 cells was assessed for cells cultured for 3, 4 and 7 days under the same conditions as used for the MTT assay. 24-well plates were then taken out of the incubator (at 37 °C) and left on bench (at 20 °C) for 15-20 min, followed by gentle agitation using 200µl micropipette (for controls as well, under identical treatments and conditions) to break cell sheets and fragments. It was noted that by days 3 and 4, most of the surfaces were fully covered with cells. By day 7, the whole surfaces were fully packed with dense cells. It was found that by following the same thermo-responsive detachment procedures as described, cells could be easily detached from these surfaces cultured under different timespans.

In summary, the MTT assays showed that cells grown on coated surfaces were just as viable as on those control surfaces. Surfaces coated at 1 and 2 mg/ml showed easy cell detachment at low, medium and high cell coverage (grown by days 3, 4 and 7), showing that cells could be easily removed below (days 3 and 4) and above full confluency (day 7). These results were supported by 3-4 repeats for each sample surface.

### Example 9 - Synthesis of poly (N-isopropylacrylamide-co-hydroxypropyl methacrylate-co-3-trimethoxysilypronylmethacrylate-co-dodecvlmethacrylate) (abbr. PNIPAAm/dodecvlmethacrylate co-polymer)

Using a process analogous to that used in Example 1, 9.05g of NIPAAm (80%mol of total), 0.72g (5%mol of total) of HPM, 1.24g (5%mol of total) of TMSPM and 2.54g of dodecylmethacrylate (10%mol of total) were reacted to provide a poly (N-isopropyl acrylamide-co-hydroxypropyl methacrylate-co-3-trimethoxysilypropyl methacrylate-co-dodecyl methacrylate) polymer with the monomer ratio of 16:1:1:2. Briefly, the above four monomers, together with 40ml absolute alcohol, were added into the three necked flask connected with a condenser. After 10 min nitrogen flow, 0.16g AIBN (1 mol% of total) was added and mixed by magnetic stirring. The reaction was carried out in a glycerol bath at 60°C for 12 hours under constant nitrogen flow with stirring. After the reaction, ethanol solvent was removed by rotary evaporation. A small amount of acetone was used to re-dissolve the sample. The product was purified by precipitation in n-hexane for three times. The final product was freeze dried for 24 hours and then stored in a refrigerator for future use.

The structure of this polymer is shown in Figure 21.

The resultant polymer was used to form temperature-responsive films using the same process as described in Example 3 above.

### Example 10 - Cell studies to compare PNIPAAm co-polymer and PNIPAAm/dodecylmethacrylate co-polymer

The cell attachment and detachment performance using 3T3 cells was assessed by seeding 3T3 cells on well containing glass coverslips coated with PNIPAAm/dodecylmethacrylate co-polymer formed at concentrations from 0.2 mg/ml to 2 mg/ml. Film coating and cell culturing were kept under the same conditions as used for assessing PNIPAAm films. The PNIPAAm/dodecylmethacrylate co-polymer films were found to support cell attachment and growth as well as cell detachment when temperature was decreased from 37 °C to 20 °C. As the films became thicker, the cells tended to dislike them and the level of adherence reduced. Instead, the cells formed large lumps. These observations were similar to those from the thicker films coated from the PNIPAAm copolymer, showing that incorporation of the dodecyl group to alter the hydrophobic nature of the copolymer and the coated films have not caused any adverse effect on the performance of the thermo-responsive film surfaces as far as cell attachment, growth and detachment are concerned.

3T3 cells grown on PNIPAAm/dodecylmethacrylate co-polymer films coated from 0.5 mg/ml solution could lead to the harvesting of large pieces of cell sheets if the cells were cultured at or above full confluence and the culture medium in the well was not disrupted, e.g., by pipetting. Detachment of cell films could be promoted and sped up if a defect in the cell film was created by scratching a hole or a line using a pipette tip. Formation of cell sheets or films provides useful building materials for fabricating 3D functional tissues useful for a range of applications including tissue engineering, drug screening and development of medical devices.

## Claims

1. A temperature-responsive co-polymer for forming a film coating on the surface of a cell culture substrate, the co-polymer comprising temperature-responsive hydrophilic monomeric units, non-temperature responsive hydrophobic and/or hydrophilic monomeric units and a cross-linking means to covalently bind the co-polymer to the substrate surface.

2. A co-polymer according to claim 1, wherein the monomeric units are substituted methacrylic, acrylic and/or bisacrylamide monomers.

3. A co-polymer according to claim 1 or claim 2, wherein the temperature-responsive monomers are selected from N-alkyl substituted acrylamides and N-alkyl substituted methacrylamides, N,N-di-alkyl substituted acrylamide and N,N-di-alkyl substituted methacrylamide or mixtures thereof.

4. A co-polymer according to any one of the preceding claims, wherein the temperature-responsive monomer is selected from N-isopropylacrylamide and N-isopropylmethacrylamide, or a mixture thereof.

5. A co-polymer according to any one of the preceding claims, wherein the hydrophobic and/or hydrophilic monomers are N-substituted acrylate or N-substituted methacrylate monomers.

6. A co-polymer according to any one of the preceding claims, wherein the cross-linking means is a functional reactive group present as part of the temperature responsive or hydrophilic/hydrophobic monomeric units or the cross-linking means is an additional monomeric constituent of the co-polymer having a functional group that reacts with functional groups present on the substrate surface.

7. A co-polymer according to any one of the preceding claims, wherein the cross-linking means is a silane cross-linking group selected from a methoxysilane group and/or an ethoxysilane group.

8. A co-polymer according to any one of the preceding claims, wherein the co-polymer is poly(N-isopropylacrylamide -co- hydroxypropyl methacrylate -co- 3-trimethoxysilypropyl methacrylate) or poly(N-isopropylacrylamide -co- hydroxypropyl methacrylate (HPM) -co- 3-trimethoxysilylpropyl methacrylate (TMSPM) -co-dodecylmethacrylate).

9. A process for the preparation of a temperature-responsive co-polymer according to claims 1 to 8, the process comprising the polymerisation of temperature-responsive monomeric units and one or more additional hydrophobic and/or hydrophilic monomeric units.

10. A substrate having a surface upon which cells can grow and divide in culture, wherein said surface is coated with a polymeric film coating formed from a temperature-responsive co-polymer as defined in any one of claims 1 to 8, and said polymeric film coating is covalently bound to the substrate surface.

11. A substrate according to claim 10, wherein the substrate is a cell culture vessel.

12. A coating solution for coating the surface of a substrate, the coating solution comprising a temperature-responsive co-polymer as defined in claims 1 to 8 dissolved in a solvent.

13. A kit of parts comprising a temperature responsive co-polymer as defined in claims 1 to 8 and a solvent for dissolving the co-polymer to form a solution which can subsequently be applied to the substrate surface.

14. A method of forming a temperature-responsive film coating on a substrate surface, said method comprising dissolving a temperature responsive co-polymer as defined in claims 1 to 8 in a solvent to form a solution, applying the solution onto the surface of the substrate, removing the solvent and annealing the co-polymer to the substrate surface.

15. A method of dislodging cells from a substrate surface which is coated with a temperature responsive co-polymer as defined in claims 1 to 8, the method comprising cooling the substrate to hydrate the temperature responsive co-polymer and thereby dislodging the cells present on the substrate surface.

16. A method of culturing cells comprising seeding cells on a substrate as defined in claims 10 or 11 in the presence of a culture medium and maintaining the temperature at an incubation temperature to allow the cells to grow and divide and then reducing the temperature to harvest the cells.

## Patentansprüche

1. Temperatursensitives Copolymer zum Bilden eines Filmüberzugs auf der Oberfläche eines Zellkultursubstrats, das Copolymer umfassend temperatursensitive hydrophile monomere Einheiten, nicht temperatursensitive hydrophobe und/oder hydrophile monomere Einheiten und ein Vernetzungsmittel, um das Copolymer an die Substratoberfläche kovalent zu binden.

2. Copolymer nach Anspruch 1, wobei die monomeren Einheiten substituierte methacrylische Monomere, acrylische Monomere und/oder Bisacrylamid-Monomere sind.

3. Copolymer nach Anspruch 1 oder Anspruch 2, wobei die temperatursensitiven Monomere gewählt sind aus N-Alkyl-substituierten Acrylamiden und N-Alkyl-substituierten Methacrylamiden, N,N-Di-Alkyl-substituierten Acrylamiden und N,N-Di-Alkyl-substituierten Methacrylamiden oder Mischungen daraus.

4. Copolymer nach einem der vorangehenden Ansprüche, wobei das temperatursensitive Monomer gewählt ist aus N-Isopropylacrylamid und N-Isopropylmethacrylamid oder Mischungen daraus.

5. Copolymer nach einem der vorangehenden Ansprüche, wobei die hydrophoben und/oder hydrophilen Monomere N-substituierte Acrylat-Monomere oder N-substituierte MethacrylatMonomere sind.

6. Copolymer nach einem der vorangehenden Ansprüche, wobei das Vernetzungsmittel eine funktionelle reaktive Gruppe ist, die als Teil des temperatursensitiven oder der hydrophilen/hydrophoben monomeren Einheiten oder des Vernetzungsmittels vorliegt, ein zusätzlicher monomerer Bestandteil des Copolymers ist, der eine funktionelle Gruppe aufweist, die mit funktionellen Gruppen reagiert, die an der Substratoberfläche vorliegen.

7. Copolymer nach einem der vorangehenden Ansprüche, wobei das Vernetzungsmittel eine vernetzende Silangruppe ist, gewählt aus einer Methoxysilangruppe und/oder einer Ethoxysilangruppe.

8. Copolymer nach einem der vorangehenden Ansprüche, wobei das Copolymer Poly(N-isopropylacrylamid-co-hydroxypropylmethacrylat-co-3-trimethoxysilylpropylmethacrylat) oder Poly(N-isopropylacrylamid-co-hydroxypropylmethacrylat (HPM)-co-3-trimethoxysilylpropylmethacrylat (TMSPM)-co-dodecylmethacrylat) ist.

9. Verfahren zur Herstellung eines temperatursensitiven Copolymers nach einem der Ansprüche 1 - 8, das Verfahren umfassend die Polymerisation der temperatursensitiven monomeren Einheiten und einer oder mehr zusätzlichen hydrophoben und/oder hydrophilen monomeren Einheiten.

10. Substrat mit einer Oberfläche, auf der Zellen in Kultur wachsen und sich teilen können, wobei die Oberfläche beschichtet ist mit einem polymeren Filmüberzug, gebildet aus einem temperatursensitiven Copolymer wie in einem der Ansprüche 1 bis 8 definiert, und der polymere Filmüberzug an die Substratoberfläche kovalent gebunden ist.

11. Substrat nach Anspruch 10, wobei das Substrat ein Zellkulturgefäß ist.

12. Beschichtungslösung zum Beschichten der Oberfläche eines Substrats, die Beschichtungslösung umfassend ein temperatursensitives Copolymer wie in den Ansprüchen 1 bis 8 definiert, gelöst in einem Lösungsmittel.

13. Kit aus Teilen, umfassend ein temperatursensitives Copolymer wie in den Ansprüchen 1 bis 8 definiert und ein Lösungsmittel zum Lösen des Copolymers, um eine Lösung zu bilden, die anschließend auf die Substratoberfläche aufgebracht werden kann.

14. Verfahren zum Bilden eines temperatursensitiven Filmüberzugs auf einer Substratoberfläche, das Verfahren umfassend das Lösen eines wie in den Ansprüchen 1 bis 8 definierten temperatursensitiven Copolymers in einem Lösungsmittel, um eine Lösung zu bilden, Aufbringen der Lösung auf die Oberfläche des Substrats, Entfernen des Lösungsmittels und Aushärten des Copolymers auf der Substratoberfläche.

15. Verfahren zum Entfernen von Zellen von einer Substratoberfläche, die mit einem wie in den Ansprüchen 1 bis 8 definierten temperatursensitiven Copolymer beschichtet ist, das Verfahren umfassend das Abkühlen des Substrats um das temperatursensitive Copolymer zu hydratisieren und dadurch Entfernen der auf der Substratoberfläche vorhandenen Zellen.

16. Verfahren zum Kultivieren von Zellen, umfassend das Plazieren von Zellen auf einem Substrat wie in einem der Ansprüche 10 oder 11 definiert in Gegenwart eines Kulturmediums und Aufrechterhalten der Temperatur bei einer Inkubationstemperatur, um den Zellen zu ermöglichen zu wachen und sich zu teilen und anschließendes Reduzieren der Temperatur zum Sammeln der Zellen.

## Revendications

1. Copolymère sensible à la température pour former un revêtement de type film sur la surface d'un substrat de culture cellulaire, le copolymère comprenant des motifs monomères hydrophiles sensibles à la température, des motifs monomères hydrophobes et/ou hydrophiles insensibles à la température et un moyen de réticulation pour lier par covalence le copolymère à la surface du substrat.

2. Copolymère selon la revendication 1, dans lequel les motifs monomères sont des monomères méthacryliques, acryliques et/ou bisacrylamide substitués.

3. Copolymère selon la revendication 1 ou la revendication 2, dans lequel les monomères sensibles à la température sont choisis parmi les acrylamides substitués par un N-alkyle et les méthacrylamides substitués par un N-alkyle, l'acrylamide substitué par un N,N-di-alkyle et le méthacrylamide substitué par un N,N-di-alkyle ou leurs mélanges.

4. Copolymère selon l'une quelconque des revendications précédentes, dans lequel le monomère sensible à la température est choisi parmi le N-isopropylacrylamide et le N-isopropyl-méthacrylamide, ou leur mélange.

5. Copolymère selon l'une quelconque des revendications précédentes, dans lequel les monomères hydrophobes et/ou hydrophiles sont des monomères d'acrylate N-substitués ou de méthacrylate N-substitués.

6. Copolymère selon l'une quelconque des revendications précédentes, dans lequel le moyen de réticulation est un groupe réactif fonctionnel présent au sein des motifs monomères sensibles à la température ou hydrophiles/hydrophobes ou le moyen de réticulation est un constituant monomère supplémentaire du copolymère ayant un groupe fonctionnel qui réagit avec les groupes fonctionnels présents sur la surface du substrat.

7. Copolymère selon l'une quelconque des revendications précédentes, dans lequel le moyen de réticulation est un groupe de réticulation silane choisi parmi un groupe méthoxysilane et/ou un groupe éthoxysilane.

8. Copolymère selon l'une quelconque des revendications précédentes, dans lequel le copolymère est un poly(N-isopropylacrylamide-co-méthacrylate d'hydroxypropyle-co-méthacrylate de 3-triméthylsilylpropyle) ou un poly(N-isopropylacrylamide-co-méthacrylate d'hydroxypropyle (HPM)-co-méthacrylate de 3-triméthoxysilylpropyle (TMSPM)-co-méthacrylate de dodécyle).

9. Procédé de préparation d'un copolymère sensible à la température selon les revendications 1 à 8, le procédé comprenant la polymérisation de motifs monomères sensibles à la température et d'un ou de plusieurs motifs monomères hydrophobes et/ou hydrophiles supplémentaires.

10. Substrat ayant une surface sur laquelle des cellules peuvent se développer et se diviser en culture, dans lequel ladite surface est revêtue d'un revêtement de type film polymère formé à partir d'un copolymère sensible à la température tel que défini dans l'une quelconque des revendications 1 à 8, et ledit revêtement de type film polymère est lié par covalence à la surface du substrat.

11. Substrat selon la revendication 10, dans lequel le substrat est un réacteur de culture cellulaire.

12. Solution de revêtement pour revêtir la surface d'un substrat, la solution de revêtement comprenant un copolymère sensible à la température tel que défini dans les revendications 1 à 8 dissous dans un solvant.

13. Kit de composants comprenant un copolymère sensible à la température tel que défini dans les revendications 1 à 8 et un solvant pour dissoudre le copolymère et former une solution qui peut ensuite être appliquée à la surface du substrat.

14. Procédé de formation d'un revêtement de type film sensible à la température sur la surface d'un substrat, ledit procédé comprenant la dissolution d'un copolymère sensible à la température tel que défini dans les revendications 1 à 8 dans un solvant pour former une solution, l'application de la solution sur la surface du substrat, l'élimination du solvant et le recuit du copolymère sur la surface du substrat.

15. Procédé de délogement de cellules de la surface d'un substrat qui est revêtu d'un copolymère sensible à la température tel que défini dans les revendications 1 à 8, le procédé comprenant le refroidissement du substrat pour hydrater le copolymère sensible à la température et ce faisant le délogement des cellules présentes sur la surface du substrat.

16. Procédé de culture de cellules comprenant l'ensemencement de cellules sur un substrat tel que défini dans les revendications 10 ou 11 en présence d'un milieu de culture et le maintien de la température à une température d'incubation pour permettre aux cellules de se développer et de se diviser, puis la réduction de la température pour récolter les cellules.
